# EUROPEAN PATENT SPECIFICATION

(11) **EP 1 246 920 B1**
(45) Date of publication and mention of the grant of the patent: **05.10.2011**
(21) Application number: 00960628.6
(22) Date of filing: 28.08.2000
(51) Int. Cl.: C12N 15/34, C07K 14/01, A61K 39/12, C12N 7/00

(54) **PREVENTION OF AFFECTIONS ASSOCIATED WITH PORCINE CIRCOVIRUS-2**
VORBEUGUNG VON MIT SCHWEINECIRCOVIRUS-2 ASSOZIERTEN KRANKHEITEN
PREVENTION D'AFFECTIONS ASSOCIEES AU CIRCOVIRUS PORCIN DE TYPE 2

(30) Priority: 31.08.1999 US 151564 P; 31.05.2000 US 583350
(43) Date of publication of application: 09.10.2002
(73) Proprietor: MERIAL, 69002 Lyon (FR); UNIVERSITY OF SASKATCHEWAN, Saskatoon, Saskatchewan S7N 5B5 (CA); The Queen's University of Belfast, Belfast BT4 3SD (GB)
(72) Inventor: ELLIS, John Albert, Saskatchewan S7N 0M3 (CA); ALLAN, Gordon, Moore, Belfast (GB); MEEHAN, Brian, Belfast BT1 3LX (GB); CLARK, Edward, Saskatchewan S7J 214 (CA); HAINES, Deborah, Saskatchewan S7N 0M3 (CA); HASSARD, Lori, Saskathewan S7K 6B5 (CA); HARDING, John, Saskatchean S0K 2A0 (CA); CHARREYRE, Catherine, Elisabeth, F-69720 Saint-Laurent de Mure (FR); CHAPPUIS, Gilles, Emile, F-69006 Lyon (FR); KRAKOWKA, George, Steve, Colombus, OH 43202 (US); AUDONNET, Jean-Christophe, Francis, F-69006 Lyon (FR); MCNEILLY, Francis, Newtownards BT3 4NH (GB)
(74) Representative: Harding, Charles Thomas
(86) International application number: PCT/EP2000/008781
(87) International publication number: WO 2001/016330

(56) References cited:
- WO-A-99/18214
- WO-A-99/29871
- ELLIS, J. ET AL.: "Reproduction of lesions of postweaning multisystemic wasting syndrome in gnotobiotic piglets." J VET DIAGN INVEST., vol. 11, no. 1, January 1999 (1999-01), pages 3-14, XP001023033 cited in the application
- "Mogelijke verwekker : porcine circovirus (PCV)" TIJDSCHR DIERGENEESKD. 1999 JUL 15-AUG 1;124(14-15):444-5, vol. 124, no. 14-15, 15 July 1999 (1999-07-15) - 1 August 1999 (1999-08-01), pages 444-445, XP001020828
- NAYAR GP, HAMEL AL, LIN L, SACHVIE C, GRUDESKI E, SPEARMAN G.: "Evidence for circovirus in cattle with respiratory disease and from aborted bovine fetuses." CAN VET J. 1999 APR;40(4):277-8, XP001035157
- LAROCHELLE R. ET AL.: "Identification and incidence of porcine circovirus in routine field cases in Quebec as determined by PCR." VET REC. , vol. 145, no. 5, 31 July 1999 (1999-07-31), pages 140-142, XP001023028
- ALLAN G.M. ET AL.: "Experimental reproduction of severe wasting disease by co-infection of pigs with porcine circovirus and porcine parvovirus" J COMP PATHOL, vol. 121, no. 1, 14 June 1999 (1999-06-14), pages 1-11, XP001020821
- BOLT A.M. ET AL.: "Non-suppurative myocarditis in piglets associated with porcine parvovirus association" J COMP PATH, vol. 117, 1997, pages 107-118, XP001034314 cited in the application
- WEST, K.H. ET AL.: "Myocarditis and abortion associated with intrauterine infection of sows with porcine circovirus 2" JOURNAL VET DIAGN INVEST, vol. 11, November 1999 (1999-11), pages 530-532, XP001020894 cited in the application
- MEEHAN, B.M. ET AL.: " Isolation and characterization of porcine circovirus 2 from cases of sow abortion and porcine dermatitis and nephropathy syndrome. " ARCHIVE OF VIROLOGY, vol. 146, no. 4, 2001, pages 835-842, XP001020414
- BOGDAN J, WEST K, CLARK E, KONOBY C, HAINES D, ALLAN G, MCNEILLY F, MEEHAN B, KRAKOWKA S, ELLIS JA.: "Association of porcine circovirus 2 with reproductive failure in pigs: a retrospective study, 1995-1998." CAN VET J. 42(7):548-50., July 2001 (2001-07), XP001023023
- O'CONNOR B. ET AL.: "Multiple porcine circovirus 2-associated abortions and reproductive in a multisite swine production unit" CAN VET J. , vol. 42, no. 7, July 2001 (2001-07), pages 551-553, XP001023022

## Description

### FIELD OF THE INVENTION

The invention relates to DNA plasmid vectors and/or compositions for reducing the viral load in the bronchial and mesenteric nodes of a pig.

### BACKGROUND OF THE INVENTION

Porcine circovirus-2 (PCV-2) was recently identified as an agent that has been consistently associated with post-weaning multisystemic wasting syndrome (PMWS) in swine populations in several parts of the world (Allan et al. 1998; Ellis et al., 1998). Isolates of PCV-2 obtained from infected pigs in several countries are virtually identical genetically, and are distinctly different from the PCV (CCL33, PCV-1) that was originally identified in the 1970's as a noncytopathic contaminant of porcine kidney (PK/15) cell line (Meehan et al. 1998; Tischer et al. 1974). Pigs with naturally acquired or experimentally induced PCV-2 infections present with progressive weight loss, tachypnea, dyspnea, and jaundice (Allan et al. 1998; Allan et al. 1999; Ellis et al. 1998; Ellis et al. 1999). Gross pathologic findings that have been directly associated with PCV-2 antigen include, lymphadenopathy, interstitial pneumonia, hepatitis and nephritis (Allan et al. 1998; Allan et al. 1999; Ellis et al. 1998; Ellis et al. 1999). See also WO-A-99 18214, WO-A-00 01409, WO-A-99 29717 and WO-A-99 29871. PCV-2 has not heretofore been directly linked to abortion or lesions in fetal pigs.

Thus, heretofore, it has not been proposed to address the issue of PCV-2-caused myocarditis, and/or abortion and/or intrauterine infection.

### OBJECTS AND SUMMARY OF THE INVENTION

It has surprisingly been found that PCV-2 is a causative agent of myocarditis, abortion and intrauterine infection, as well as post-weaning multisystemic wasting syndrome.

By definition, a PCV-2 immunogen is intended to encompass live attenuated or inactivated PCV-2, or subunit(s) from PCV-2 obtained by *in vitro* expression or by extraction, or fragment(s) comprising at least one epitope of interest which can be obtained by chemical synthesis or by *in vitro* recombinant expression, as well as recombinant vector(s) comprising and expressing *in vivo* sequence(s) or fragment(s) or epitope(s) of PCV-2 genome as herein disclosed or as in documents cited or referenced herein.

A similar definition applies for an immunogen of another porcine pathogen as disclosed herein.

By definition, an immunogenic composition elicits an immunogenic response-local or systemic. A vaccine composition elicits a local or systemic protective response. The term "immunogenic composition" include a "vaccine composition" (as the former term can be protective composition).

According to a first aspect of the present invention there is provided a first DNA plasmid vector comprising a first nucleotide sequence having at least 95% identity with ORF13 of PCV-2 strain Imp1010, for use in reducing the viral load of PCV-2 in the bronchial and mesenteric nodes of a pig.

According to a second aspect of the present invention there is provided an immunogenic composition for use in reducing the viral load of PCV-2 in the bronchial and mesenteric nodes of a pig, wherein said composition comprises:
a) a first DNA plasmid vector comprising a first nucleotide sequence having at least 95% identity with ORF13 of PCV-2 strain Imp1010, and
b) a pharmaceutically or veterinarily acceptable carrier and/or vehicle and/or excipient and/or adjuvant.

According to a third aspect of the present invention there is provided use of:
a) a first DNA plasmid vector comprising a first nucleotide sequence having at least 95% identity with ORF13 of PCV-2 strain Imp1010 and
b) a pharmaceutically or veterinarily acceptable carrier and/or vehicle and/or excipient and/or adjuvant
   in the preparation of an immunogenic composition for use in reducing the viral load of PCV-2 in the bronchial and mesenteric nodes of a pig.

According to a fourth aspect of the present invention there is provided a first DNA plasmid vector comprising a first nucleotide sequence having at least 95% identity with ORF1 3 of PCV-2 strain Imp1010, and a second DNA plasmid vector comprising a second nucleotide sequence having at least 95% identity with ORF4 of PCV-2 strain Imp1010, for use in reducing the viral load of PCV-2 in the bronchial and mesenteric nodes of a pig.

According to a fifth aspect of the present invention there is provided an immunogenic composition for use in reducing the viral load of PCV-2 in the bronchial and mesenteric nodes of a pig, wherein said composition comprises:
a) a first DNA plasmid vector comprising a first nucleotide sequence having at least 95% identity with ORF13 of PCV-2 strain Imp1010,
b) a second DNA plasmid vector comprising a second nucleotide sequence having at least 95% identity with ORF4 of PCV-2 strain Imp1010, and
c) a pharmaceutically or veterinarily acceptable carrier and/or vehicle and/or excipient and/or adjuvant

According to a sixth aspect of the present inivention there is provided use of:
a) a first DNA plasmid vector comprising a first nucleotide sequence having at least 95% identity with ORF13 of PCV-2 strain Imp1010,
b) a second DNA plasmid vector comprising a second nucleotide sequence having at least 95% identity with ORF4 of PCV-2 strain Imp1010, and
c) a pharmaceutically or veterinarily acceptable carrier and/or vehicle and/or excipient and/or adjuvant
   in the preparation of an immunogenic composition for use in reducing the viral load of PCV-2 in the bronchial and mesenteric nodes of a pig.

According to a seventh aspect of the present invention there is provided the first plasmid for use according to the first aspect of the present invention; or the immunogenic composition for use according to the second aspect of the present invention; or the use of the first plasmid according to the third aspect of the present invention; or the first and second plasmids for use according to the fourth aspect of the present invention; or the immunogenic composition for use according to the fifth aspect of the present invention; or the use of a first and second plasmids according to the sixth aspect of the present invention;
wherein said first nucleotide sequence is ORF13 of PCV-2 strain Imp1010.

According to an eighth aspect of the present invention there is provided the first and second plasmids for use according to the fourth or seventh aspect of the present invention; or the immunogenic composition for use according to the fifith or seventh aspect of the present invention; or the use of a first and second plasmid according to the sixth and seventh aspect of the present invention;
wherein said second nucleotide sequence is ORF4 of PCV-2 strain Imp1010.

We describe methods and/or compositions for the prevention and/or treatment of PCV-2-caused myocarditis, and/or abortion and/or intrauterine infection, as well as post-weaning multisystemic wasting syndrome and/or pathologic sequelae including but not limited to post-weaning multisystemic wasting syndrome; and, methods for formulating such compositions (which compositions can also include a porcine parvovirus (PPV) immunogen) and uses of a PCV-2 immunogen for formulating such compositions.

We describe the use of PCV-2 immunogens to prepare compositions for prevention and/or treatment of PCV-2 caused myocarditis, and/or abortion, and/or intrauterine infection.

We describe the isolation and characterisation of new PCV-2 strains identified 1103 (1103/1 P.2) and 1121 (1121/1 P.1), and their uses to produce immunogens, in relation with PCV-2-caused myocarditis, and/or abortion and/or intrauterine infection, as well as post-weaning multisystemic wasting syndrome and/or pathologic sequelae associated therewith.

We describe inoculation of female pigs (e.g., sows, gilts) with a composition comprising a (at least one) PCV-2 immunogen (which composition can also include an immunogen from porcine parvovirus) prior to breeding, and/or prior to serving, and/or during gestation (or pregnancy); and/or prior to the perinatal period or farrowing, and/or repeatedly over a lifetime, to prevent myocarditis and/or abortion and/or intrauterine infection associated with PCV-2, as well as post-weaning multisystemic wasting syndrome and other pathologic sequelae associated with PCV-2; or, to elicit an immunogenic or protective response against PCV-2 and thereby prevent post-weaning multisystemic wasting
syndrome and/or myocarditis and/or abortion and/or intrauterine infection associated with porcine circovirus-2 and/or other pathologic sequelae associated with PCV-2.

Advantageously, at least one inoculation is done before serving. It is also advantageously followed by an inoculation to be performed during gestation, e.g., at about mid-gestation (at about 6-8 weeks of gestation) and/or at the end of gestation (at about 11-13 weeks of gestation). Thus, an advantageous regimen is an inoculation before serving and a booster inoculation during gestation. Thereafter, there can be reinoculation before each serving and/or during gestation at about mid-gestation (at about 6-8 weeks of gestation) and/or at the end of gestation (at about 11-13 weeks of gestation). Preferably, reinoculation can be during gestation only.

In another preferred embodiment, piglets, such as piglets from vaccinated females (e.g., inoculated as herein discussed), are inoculated within the first weeks of life, e.g., inoculation at one and/or two and/or three and/or four and/or five weeks of life. More preferably, piglets are first inoculated within the first week of life or within the third week of life (e.g., at the time of weaning). Even more advantageous, such piglets are then boosted two (2) to four (4) weeks later (after being first inoculated). Thus, both offspring, as well as female pig (e.g., sow, gilt) can be administered compositions of the invention and/or can be the subject of performance of methods of the invention.

We describe immunogenic or vaccine compositions comprising immunogen(s) from PCV-2 strain(s) 1103 and/or 1121, for preventing or treating myocarditis and/or abortion and/or intrauterine infection associated with porcine circovirus-2, as well as post-weaning multisystemic wasting syndrome and other pathologic sequelae associated with PCV-2.

We further describe uses of a PCV-2 immunogen to formulate an immunogenic or vaccine composition for preventing or treating myocarditis and/or abortion and/or intrauterine infection associated with porcine circovirus-2, as well as post-weaning multisystemic wasting syndrome and other pathologic sequelae associated with PCV-2.

Further still, we describe an immunogenic or vaccine composition for the prevention and/or treatment of PCV-2-caused myocarditis, and/or abortion and/or intrauterine infection and/or post-weaning multisystemic wasting syndrome comprising a pharmaceutically or veterinarily acceptable carrier and/or vehicle and/or excipient and/or adjuvant, and a PCV-2 immunogen.

We describe that the composition can additionally include at least one immunogen from at least one additional pig pathogen, e.g.: Porcine Reproductive and Respiratory Syndrome (PRRS), Mycoplasma hyopneumoniae, Actinobacillus pleuropneumoniae, *E*. *coli*, Bordetella bronchiseptica, Pasteurella multocida, Erysipelothrix rhusiopathiae, Pseudorabies, Hog cholera, Swine Influenza, and Porcine Parvovirus (PPV). Thus, vector-based compositions can include at least one immunogen from at least one additional pig pathogen, such as a vector expressing a sequence from this pathogen, wherein the vector can also be the vector expressing the PCV-2 immunogen. The vector expressing a PCV-2 sequence can comprise a PCV-2 sequence or fragment; and the invention comprehends such nucleic acid molecules, vectors containing them, compositions comprising such nucleic acid molecules or vector expression products from such nucleic acid molecules, compositions comprising such expression products, probes or primers for such nucleic acid molecules, and methods for making and using any or all of the foregoing.

According to the present invention, the vector comprises a DNA vector plasmid. We also describe the following vectors: a bacteria such as an *E. coli*, a virus such as baculovirus, a herpesvirus including pig herpes viruses, including Aujeszky's disease virus, an adenovirus including a porcine adenovirus, a poxvirus, including a vaccinia virus, an avipox virus, a canarypox virus, a racoonpox and a swinepox virus, and the like. The vector-based compositions can comprise a vector that contains and expresses an ORF selected from ORFs 4 and/or 13, of a PCV-2, advantageously of any one of the PCV-2 strains identified herein and in particular of strains 1103 and/or 1121. And, the immunogen in compositions (either PCV-2 and/or from another pig pathogen) can be recombinantly produced.

The word plasmid is intended to include any DNA transcription unit in the form of a polynucleotide sequence comprising the PCV sequence to be expressed. Advantageously, the plasmid includes elements necessary for its expression; for instance, expression *in vivo.* The circular plasmid form, supercoiled or otherwise, is advantageous; and, the linear form is also included within the scope of the invention. The plasmid immunogenic or vaccine composition can be administered by way of a gene gun, intradermally via an needleless injector, subcutaneously or intramuscularly, or by mucosal route, or by any other means that allows for expression *in vivo,* and advantageously an immunogenic or protective response.

It is noted that the expression product generated by vectors or recombinants we describe optionally can also be isolated and/or purified from infected or transfected cells; for instance, to prepare compositions for administration to pigs; however, in certain instances, it may be advantageous not to isolate and/or purify an expression product from a cell; for instance, when the cell or portions thereof enhance the immunogenic effect of the polypeptide.

And, techniques for protein purification and/or isolation are known and can be used, without undue experimentation, to purify and/or isolate recombinant or vector expression products and/or subunits of PCV-2 and/or other pig pathogens, in the practice of the invention; such techniques, in general, can include: precipitation by taking advantage of the solubility of the protein of interest at varying salt concentrations, precipitation with organic solvents, polymers and other materials, affinity precipitation and selective denaturation; column chromatography, including high performance liquid chromatography (HPLC), ion-exchange, affinity, immunoaffinity or dye-ligand chromatography; immunoprecipitation, gel filtration, electrophoretic methods, ultrafiltration and isoelectric focusing, and their combinations, *inter alia.*

We describe methods for the prevention and/or treatment of porcine circovirus-2 (PCV-2)-caused myocarditis, and/or abortion and/or intrauterine infection and/or post-weaning multisystemic wasting syndrome and/or other pathologic sequelae associated with PCV-2 comprising inducing an immunogenic or protective response against PCV-2 in a pig comprising administering to the pig an aforementioned or herein disclosed composition.

Thus, we describe a method for the prevention and/or treatment of porcine circovirus-2 (PCV-2)-caused myocarditis, and/or abortion and/or intrauterine infection and/or post-weaning multisystemic wasting syndrome and/or other pathologic sequelae associated with PCV-2 comprising inducing an immunogenic or protective response against PCV-2 in a pig comprising administering to the pig a composition comprising a pharmaceutically or veterinarily acceptable carrier or excipient or vehicle, with preferably an adjuvant, and an active agent comprising a PCV-2 immunogen. The method can be for the prevention of PCV-2-caused myocarditis and/or abortion and/or intrauterine infection comprising administering a composition comprising a acceptable carrier and a PCV-2 immunogen. The method can involve a composition that is a subunit immunogenic composition. The method can involve the composition additionally including at least one immunogen from at least one additional pig pathogen, including a vector expressing such an immunogen or epitope; e.g., the at least one additional pig pathogen can be selected from the group consisting of PRRS, Mycoplasma hyopneumoniae, Actinobacillus pleuropneumoniae, *E. coli*, Pseudorabies, Hog cholera, Bordetella bronchiseptica, Pasteurella multocida, Erysipelothrix rhusiopathiae, Swine Influenza, and PPV and combinations thereof. The method can involve a vector that is the DNA vector plasmid for use in the invention or a described vector that is, a bacteria such as an *E*. *coli,* a virus such as baculovirus, a herpesvirus including Aujeszky's disease virus, an adenovirus including a porcine adenovirus, a poxvirus, including a vaccinia virus, an avipox virus, a canarypox virus, and a swinepox virus, and the like. The method can involve a vector-based composition additionally including at least one sequence, fragment or epitope from at least one additional pig pathogen, or a vector expressing such a sequence, fragment or epitope, wherein the vector can also be the vector expressing the PCV-2 sequence, fragment or epitope. The method can involve a vector that contains and expresses an ORF selected from the group consisting of ORFs 1 to 13, e.g., an ORF selectred from ORFs 4, 7, 10 and 13; preferably ORFs 4 and/or 13. The method can also involve an immunogen-based composition wherein one or more of the immunogen(s) is recombinantly produced In this method, females and/or piglets are preferably inoculated as described above.

In another embodiment, the invention involves a method for preparing any of the aforementioned or herein disclosed compositions comprising admixing the pharmaceutically or veterinarily acceptable carrier and possibly the adjuvant, and the DNA plasmid vector. We describe a method that can further include transfecting or infecting a cell or host with a recombinant vector that contains DNA encoding a PCV-2 immunogen and expresses that immunogen; and optionally purifying and/or isolating the immunogen from the cell. Similarly the method can include isolating and/or purifying a PCV-2 immunogen from PCV-2, or isolating PCV-2 from a sample.

We also describe a kit for preparing any of the aforementioned or herein disclosed compositions or for performing any of the aforementioned or herein disclosed methods comprising in a first container the pharmaceutically or veterinarily acceptable carrier or vehicle or excipient and in a second container the active agent comprising the PCV-2 immunogen, wherein the first and second containers are optionally packaged together, and the kit optionally includes instructions for admixture of ingredients of the composition and/or administration of the composition.

We further describe administering any of the aforementioned or herein disclosed compositions to male and/or female pigs; to prevent transmission of PCV-2 and prevent or treat or control myocarditis and/or abortion and/or intrauterine infection associated with porcine circovirus-2, as well as post-weaning multisystemic wasting syndrome and other pathologic sequelae associated with PCV-2.

Administration is preferably done as described above.

The term "comprising" in this disclosure can mean "including" or can have the meaning commonly given to the term "comprising" in U.S. Patent Law.

Other aspects of the invention are described in or are obvious from (and within the ambit of the invention) the following disclosure.

### DETAILED DESCRIPTION

Porcine circovirus-2 (PCV-2) is an agent associated with post-weaning multisystemic wasting syndrome (PMWS) in swine populations. As shown in Reference Examples 1 and 2, the potential spectrum of disease associated with PCV-2 is expanded by evidence of vertical transmission and associated reproductive failure.

In particular, Example 1 shows that PCV-2 was isolated from a litter of aborted piglets from a farm experiencing late term abortions and stillbirths. Severe, diffuse myocarditis was present in one piglet associated with extensive immunohistochemical staining for PCV-2 antigen Variable amounts of PCV-2 antigen were also present in liver, lung and kidney of multiple fetuses. The presence of other agents that have been associated with fetal lesions and abortion in swine including porcine parvovirus, porcine reproductive respiratory syndrome virus, encephalomyocarditis virus and enterovirus could not be established.

More in particular, Reference Example 2 shows that tissues obtained from 30 high health herds over a four-year period, and tested in routine cases of abortion or reproductive failure, were positive for PCV-2 in two submissions involving several stillborn piglets and non-viable neonates presenting with severe diffuse myocarditis, cardiac hypertrophy and evidence of chronic passive congestion. The two positive submissions were from the same farm, but occurred at two different times. The presence of PCV-2 in the hearts and other tissues of affected piglets was confirmed by immunohistochemistry and virus isolation. Failure to detect porcine circoviruses in cases of reproductive failure prior to 1999 in areas of endemic infections supports the view that reproductive disease is a new clinical manifestation of PCV-2 infection, and further suggests that sexual, as well as vertical, modes of transmission are responsible for viral dissemination in the pig population.

Accordingly, inoculation of pigs, e.g., female pigs, such as sows or gilts, with a composition including at least one PCV-2 immunogen (e.g. from at least one strain chosen among strains Imp1008, Imp1010, Imp999, Imp1011-48285, Imp1011-48121, 1103 and 1121) (which composition can also include at least one immunogen from at least one other porcine pathogen such as at least one porcine parvovirus, wherein when a vector is used the vector can co-express both the PCV-2 immunogen(s) and the at least one immunogen of the at least one other porcine pathogen, e.g., PPV immunogen(s), *inter alia*, in particular in a schedule of immunization as described above, can prevent myocarditis and/or abortion and/or intrauterine infection associated with PCV-2, as well as post-weaning multisystemic wasting syndrome and other pathologic sequelae associated with PCV-2.

Thus, we describe methods and compositions using PCV-2 immunogen for preventing myocarditis and/or abortion and/or intrauterine infection associated with porcine circovirus-2, as well as post-weaning multisystemic wasting syndrome and other pathologic sequelae associated with PCV-2. In particular, immunogen from strain 1103 and/or strain 1121 is useful for methods and compositions using PCV-2 immunogen for preventing myocarditis and/or abortion and/or intrauterine infection associated with porcine circovirus-2.

We describe the use of any known PCV-2 strain or immunogen therefrom to formulate an immunogenic or vaccine composition for preventing or treating myocarditis and/or abortion and/or intrauterine infection associated with PCV-2, as well as post-weaning multisystemic syndrome and other pathologic sequelae associated with PCV-2 (these strains include strains Imp1008, Imp1010, Imp999, Imp1011-48285, Imp1011-48121, 1103 and 1121), in particular when the composition is intended to be administered to piglets such as to piglets from vaccinated females and more particularly to female pigs, in particular to pregnant females or females which will be subjected to serving; and more particularly according to the inoculation schemes define above.

More particularly, the use is to formulate such compositions intended to prevent or treat myocarditis and/or abortion and/or intrauterine infection associated with PCV-2, in particular when the composition is intended to be administered to piglets such as to piglets from vaccinated females and more particularly to female pigs in particular to pregnant females or females which will be subjected to serving; and more particularly according to the inoculation schemes defined above.

The at least one immunogen from at least one other porcine pathogen can be as used in known porcine vaccines or immunogenic compositions, or as in WO 98/03658.

Compositions for use in the invention can be prepared in accordance with standard techniques well known to those skilled in the veterinary or pharmaceutical or arts. Such compositions can be administered in dosages and by techniques well known to those skilled in the veterinary arts taking into consideration such factors as the age, sex, weight, condition and particular treatment of the pig, and the route of administration. The compositions can be administered alone, or can be co-administered or sequentially administered with other compositions described herein (e.g., other compositions comprising a PCV-2 immunogen) or with other prophylactic or therapeutic compositions (e.g., other porcine immunogenic or vaccine compositions). Thus, the invention also provides multivalent or "cocktail" or combination compositions and methods employing them. In this regard, reference is made to U.S. Patent No. 5,843,456 directed to rabies compositions and combination compositions and uses thereof.

Compositions for use in the invention may be used for parenteral or mucosal administration, preferably by intradermal or intramuscular routes. In particular for intradermal route, injection can be done using a needleless injector. When mucosal administration is used, it is possible to use oral, nasal, or ocular routes.

In such compositions the immunogen(s) may be in a mixture with a suitable carrier, diluent, or excipient such as sterile water, physiological saline, glucose or the like, and/or preferably with an adjuvant. The compositions can also be lyophilized or frozen. The compositions can contain auxiliary substances such as pH buffering agents, adjuvants, preservatives, polymer excipients used for mucosal routes, and the like, depending upon the route of administration and the preparation desired.

Standard texts, such as "REMINGTONS PHARMACEUTICAL SCENCE", 17th edition, 1985, may be consulted to prepare suitable preparations, without undue experimentation. Suitable dosages can also be based upon the text herein and documents cited herein.

Adjuvants are substances that enhance the immune response to immunogens. Adjuvants, can include aluminum hydroxide and aluminum phosphate, saponins e.g., Quil A, water-in-oil emulsion, oil-in-water emulsion, water-in-oil-in-water emulsion. The emulsion can be based in particular on light liquid paraffin oil (European Pharmacopea type); isoprenoid oil such as squalane or squalene; oil resulting from the oligomerization of alkenes, in particular of isobutene or decene; esters of acids or of alcohols containing a linear alkyl group, more particularly plant oils, ethyl oleate, propylene glycol di (caprylate/caprate), glyceryl tri (caprylate/caprate) or propylene glycol dioleate; esters of branched fatty acids or alcohols, in particular isostearic acid esters. The oil is used in combination with emulsifiers to form the emulsion. The emulsifiers are preferably nonionic surfactants, in particular esters of sorbitan, of mannide (e.g. anhydromannitol oleate), of glycol, of polyglycerol, of propylene glycol and of oleic, isostearic, ricinoleic or hydroxystearic acid, which are optionally ethoxylated, and polyoxypropylene-polyoxyethylene copolymer blocks, in particular the Pluronic®; products, especially L121. See Hunter et al., The Theory and Practical Application of Adjuvants (Ed. Stewart-Tull, D. E. S.). John Wiley and Sons, NY, pp51-94 (1995) and Todd et al., Vaccine 15:564-570 (1997).

For example, it is possible to use the SPT emulsion described on page 147 of "Vaccine Design, The Subunit and Adjuvant Approach" edited by M. Powell and M. Newman, Plenum Press, 1995, and the emulsion MF59 described on page 183 of this same book.

For example the adjuvant-containing vaccine is prepared in the following way: 67% v/v of aqueous phase comprising the immunogen are emulsified in 2,3% w/v of anhydromannitol oleate, 2,6% w/v of oleic acid ethoxylated with 11 EO (ethylene oxide) and 28,1% v/v of light liquid paraffin oil (European Pharmacopea type) with the aid of an emulsifying turbomixer.

An alternative method for preparing the emulsion consists in emulsifying, by passages through a high-pressure homogenizer, a mixture of 5% w/v squalane, 2.5% w/v Pluronic® L121, 0.2% w/v of an ester of oleic acid and of anhydrosorbitol ethoxylated with 20 EO, 92.3% v/v of the aqueous phase comprising the immunogen.

It is also possible to formulate with synthetic polymers (e.g., homo- and copolymers of lactic and glycolic acid, which have been used to produce microspheres that encapsulate immunogens, see Eldridge et al., Mol. Immunol. 28:287-294 (1993), e.g., biodegradable microspheres), with cytokines such as IL-2 and IL-12 (*see*, *e.g.*, U.S. Patent No. 5,334,379), and GMCSF, advantageously porcine GMCSF (granulocyte macrophage-colony stimulating factor, *see*, *generally*, U.S. Patents Nos. 4,999,291 and 5,461,663, *see also* Clark et al., Science 1987,230:1229; Grant et al., Drugs, 1992, 53:516), *inter alia.* Certain adjuvants can be expressed *in vivo* with immunogen(s) and/or epitope(s); e.g., cytokines, GMCSF (*see, e.g.,* Inumaru and Takamatsu, Immunol. Cell. Biol., 1995, 73:474-76 concerning a plasmid encoding and expressing porcine GM-CSF).

A further instance of an adjuvant is a compound chosen from the polymers of acrylic or methacrylic acid and the copolymers of maleic anhydride and alkenyl derivative. Advantageous adjuvant compounds are the polymers of acrylic or methacrylic acid which are cross-linked, especially with polyalkenyl ethers of sugars or polyalcohols. These compounds are known by the term carbomer (Phameuropa Vol. 8, No. 2, June 1996). Persons skilled in the art can also refer to U.S. Patent No. 2,909,462 which describes such acrylic polymers cross-linked with a polyhydroxylated compound having at least 3 hydroxyl groups, preferably not more than 8, the hydrogen atoms of at least three hydroxyls being replaced by unsaturated aliphatic radicals having at least 2 carbon atoms. The preferred radicals are those containing from 2 to 4 carbon atoms, e.g. vinyls, allyls and other ethylenically unsaturated groups. The unsaturated radicals may themselves contain other substituents, such as methyl. The products sold under the name Carbopolt® (BF Goodrich, Ohio, USA) are particularly appropriate. They are cross-linked with an allyl sucrose or with allyl pentaerythritol. Among them, there may be mentioned Carbopol® 974P, 934P and 971 P. Among the copolymers of maleic anhydride and alkenyl derivative, the copolymers EMA® (Monsanto) which are copolymers of maleic anhydride and ethylene, linear or cross-linked, for example cross-linked with divinyl ether, are preferred. Reference may be made to J. Fields et al., Nature, 186: 778-780,4 June 1960.

From the point of view of their structure, the polymers of acrylic or methacrylic acid and the copolymers EMA® are preferably formed of basic units of the following formula: 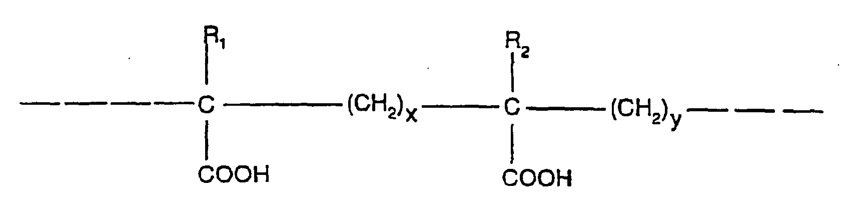 in which:
R₁, and R₂, which are identical or different, represent H or CH₃;
x = 0 or 1, preferably x = 1; and
y = 1 or 2, with x+y = 2.

For the copolymers EMA®, x = 0 and y = 2. For the carbomers, x = y =1.

The dissolution of these polymers in water leads to an acid solution that will be neutralized, preferably to physiological pH, in order to give the adjuvant solution into which the immunogenic, immunological or vaccine composition itself will be incorporated. The carboxyl groups of the polymer are then partly in COO⁻ form. Preferably, a solution of adjuvant according to the invention, especially of carbomer, is prepared in distilled water, preferably in the presence of sodium chloride, the solution obtained being at acidic pH. This stock solution is diluted by adding it to the desired quantity (for obtaining the desired final concentration), or a substantial part thereof, of water charged with NaCl, preferably physiological saline (NaCL 9 g/l) all at once in several portions with concomitant or subsequent neutralization (pH 7.3 to 7.4), preferably with NaOH. This solution at physiological pH will be used as it is for mixing with the vaccine, which may be especially stored in freeze-dried, liquid or frozen form.

The polymer concentration in the final vaccine composition can be 0.01% to 2% w/v, e.g., 0.06 to 1% w/v, such as 0.1 to 0.6% w/v.

From this disclosure and the knowledge in the art, the skilled artisan can select a suitable adjuvant, if desired, and the amount thereof to employ in an immunogenic composition for use in the invention, without undue experimentation.

We describe that the immunogenic compositions for use in the invention may be associated to at least one live attenuated, inactivated, or sub-unit vaccine, or recombinant vaccine (e.g. poxvirus as vector or DNA plasmid) expressing at least one immunogen or epitope of interest from at least one another pig pathogen.

Compositions in forms for various administration routes are envisioned as being for use in the invention. And again, the effective dosage and route of administration are determined by known factors, such as age, sex, weight and other screening procedures which are known and do not require undue experimentation. Dosages of each active agent can be as in herein cited documents and/or can range from one or a few to a few hundred or thousand micrograms, e.g., 1 µg to 1 mg, for a subunit immunogenic composition; and, we describe 10⁴ to 10¹⁰ TCID₅₀ advantageously 10⁶ to 10⁸ TCID₅₀ for an inactivated (titre before inactivation) immunogenic, or vaccine composition. For a described live attenuated immunogenic or vaccine composition, the dose can be between 10¹ and 10⁸ TCID₅₀ advantageously 10³ and 10⁶ TCID₅₀.

Recombinants or vectors can be administered in a suitable amount to obtain *in vivo* expression corresponding to the dosages described herein and/or in herein cited documents. For instance, we describe suitable ranges for viral suspensions can be determined empiracally. The described viral vector or recombinant can be administered to a pig or infected or transfected into cells in an amount of about at least 10³ pfu; more preferably about 10⁴ pfu to about 10¹⁰ pfu, e.g., about 10⁵ pfu to about 10⁹ pfu, for instance about 10⁶ pfu to about 10⁸ pfu, per dose, e.g. of about 2 ml. And, if more than one gene product is expressed by more than one recombinant, each recombinant can be administered in these amounts; or, each recombinant can be administered such that there is, in combination, a sum of recombinants comprising these amounts.

In plasmid compositions employed in the invention, dosages can be as described in documents cited herein or as described herein. For instance, suitable quantities of each plasmid DNA in plasmid compositions can be 1 µg to 2 mg, preferably 50 µg to 1 mg. Documents cited herein regarding DNA plasmid vectors may be consulted by the skilled artisan to ascertain other suitable dosages for DNA plasmid vector compositions of the invention, without undue experimentation.

However, the dosage of the composition(s), concentration of components therein and timing of administering the composition(s), which elicit a suitable immunologenic response, can be determined by methods such as by antibody titrations of sera, e.g., by ELISA and/or seroneutralization assay analysis and/or by vaccination challenge evaluation in pig. Such determination do not require undue experimentation from the knowledge of the skilled artisan, this disclosure and the documents cited herein. And, the time for sequential administrations can be likewise ascertained with methods ascertainable from this disclosure, and the knowledge in the art, without undue experimentation.

The PCV-2 immunogen can be obtained from PCV-2 or can be obtained from *in vitro* recombinant expression of PCV-2 gene(s) or portions or epitopes thereof. Methods for making and/or using vectors (or recombinants) for expression can be by or analogous to the methods disclosed in: U.S. Patent Nos. 4,603,112, 4,769,330, 5,174,993, 5,505,941, 5,338,683, 5,494,807, 4,722,848, 5,942,235, 5,364,773, 5,762,938, 5,770,212, 5,942,235, 5,756,103, 5,766,599, 6,004,777, 5,990,091, 6,033,904, 5,869,312, 5,382,425, PCT publications WO 94/16716, WO 96/39491, WO 95/30018, Paoletti, "Applications of pox virus vectors to vaccination: An update," PNAS USA 93:11349-11353, October 1996, Moss, "Genetically engineered poxviruses for recombinant gene expression, vaccination, and safety," PNAS USA 93:11341-11348, October 1996, Smith et al., U.S. Patent No. 4,745,051 (recombinant baculovirus), Richardson, C.D. (Editor), Methods in Molecular Biology 39, "Baculovirus Expression Protocols" (1995 Humana Press Inc.), Smith et al., "Production of Huma Beta Interferon in Insect Cells Infected with a Baculovirus Expression Vector," Molecular and Cellular Biology, Dec., 1983, Vol. 3, No. 12, p. 2156-2165; Pennock et al., "Strong and Regulated Expression of Escherichia coli B-Galactosidase in Infect Cells with a Baculovirus vector, "Molecular and Cellular Biology Mar. 1984, Vol. 4, No. 3, p. 399-406; EPA 0 370 573, U.S. application Serial No. 920,197, filed October 16,1986, EP Patent publication No. 265785, U.S. Patent No. 4,769,331 (recombinant herpesvirus), Roizman, "The function of herpes simplex virus genes: A primer for genetic engineering of novel vectors, "PNAS USA 93:11307-1131.2, October 1996, Andreansky et al., "The application of genetically engineered herpes simplex viruses to the treatment of experimental brain tumors, "PNAS USA 93:11313-11318, October 1996, Robertson et al. "Epstein-Barr virus vectors for gene delivery to B lymphocytes, "PNAS USA 93:11334-11340, October 1996, Frolov et al., "Alphavirus-based expression vectors: Strategies and applications, "PNAS USA 93:11371-11377, October 1996, Kitson et al., J. Virol. 65, 3068-3075, 1991; U.S. Patent Nos. 5,591,439, 5,552,143, WO 98/00166, allowed U.S. applications Serial Nos. 08/675,556, and 08/675,566 both filed July 3, 1996 (recombinant adenovirus), Grunhaus et al., 1992, "Adenovirus as cloning vectors, "Seminars in Virology (Vol. 3) p. 237-52,1993, Ballay et al. EMBO Journal, vol. 4, p. 3861-65, Graham, Tibtech 8, 85-87, April, 1990, Prevec et al., J. Gen Virol. 70, 429-434, PCT WO91/11525, Felgner et al. (1994), J. Biol. Chem. 269, 2550-2561, Science, 259: 1745-49,1993 and McClements et al., "Immunization with DNA vaccines encoding glycoprotein D or glycoprotein B, alone or in combination, induces protective immunity in animal models of herpes simplex virus-2 disease," PNAS USA 93:11414-11420, October 1996, and U.S. Patents Nos 5,591,639, 5,589,466, and 5,580,859, as well as WO-A-90 11092, WO-A-93 19183, WO-A-94 21797, WO-A-95 11307, WO-A-95 20660, Tang et al., Nature 356, 152-154, 1992, and Furth et al. Analytical Biochemistry, 205, 365-368, 1992, relating to DNA expression vectors, *inter alia*. See also WO 98/33510; Ju et al., Diabetologia, 41:736-739, 1998 (lentiviral expression system); Sanford et al., U.S. Patent No. 4,945,050; Fischbach et al. (Intracel), WO 90/01543; Robinson et al., seminars in IMMUNOLOGY, vol. 9, pp. 271-283 (1997) (DNA vector systems); Szoka et al., U.S. Patent No. 4,394,448 (method of inserting DNA into living cells); McCormick et al., U.S. Patent No. 5,677,178 (use of cytopathic viruses); and U.S. Patent No. 5,928,913 (vectors for gene delivery), as well as other documents cited herein. We describe that a viral vector, for instance, selected from pig herpes viruses, such as Aujeszky's disease virus, porcine adenovirus, poxviruses, especially vaccinia virus, avipox virus, canarypox virus, and swinepox virus are advantageously employed, as well as DNA plasmid vectors employed in the practice of the invention.

The expression product from the PCV-2 gene(s) or portions thereof can be useful for generating antibodies such as monoclonal or polyclonal antibodies that are useful for diagnostic purposes. Similarly, expression product(s) from the PCV-2 gene(s) or portions thereof can be useful in diagnostic applications.

Further, one skilled in the art can determine an epitope of interest in a PCV-2 immunogen, or in an immunogen of another porcine pathogen, without undue experimentation, from the disclosure herein and the knowledge in the art; *see*, *e.g.*, WO 98/40500, regarding general information for determining an epitope of interest or an epitopic region of a protein, *inter alia.*

We describe that advantageous immunogenic or vaccine compositions are: An immunogenic or vaccine composition, collected from a cell culture *in vitro* which has been infected with a purified preparation of PCV-2, such as a purified preparation of porcine circovirus selected from the group consisting of the preparations deposited at the ECACC (European Collection of Cell Cultures, Centre for Applied Microbiology & Research, Salisbury, Wiltshire SP4 OJG, UK), under the following references: accession No. V97100219 (strain Imp. 1008), No. V97100218 (strain Imp. 1010) and accession No. V97100217 (strain Imp. 999) deposited October 2,1997, accession No. V98011608 (strain Imp. 1011-48285) and No. V98011609 (strain Imp. 1011-48121) deposited January 16, 1998, accession No. 00012710 (strain 1103) and No. 00012709 (strain 1121) deposited February 2, 2000, or an immunogenic or vaccine composition comprised of porcine circovirus produced on, and isolated from cell culture *in vitro,* these cells having been infected with a porcine circovirus capable of being isolated from a physiological sample or from a tissue sample, especially lesions, from a pig having the PMWS syndrome, e.g., such a composition wherein the porcine circovirus is produced on, and isolated from a pig kidney cell line, for instance, produced on, and isolated from PK/15 cells free from contamination with PCV-1; or such a composition comprising or prepared from a culture extract or supernatant, collected from a cell culture *in vitro* which have been infected with a such a circovirus. Thus, we describe that porcine circovirus can be an immunogen.

We describe the vaccine or immunogenic composition can comprise PCV-2 immunogens and/or immunogens of several porcine circoviruses (including PCV-2 or several strains of PCV-2, and including PCV-1), as well as optionally additional immunogens from another pig pathogen; e.g, PRRS, Mycoplasma hyopneumoniae, Actinobacillus pleuropneumoniae, *E*. *coli,* Pseudorabies, Hog cholera, Bordetella bronchiseptica, Pasteurella multocida, Erysipelothrix rhusiopathiae, Swine Influenza, PPV (*see also* WO-A-00 01409).

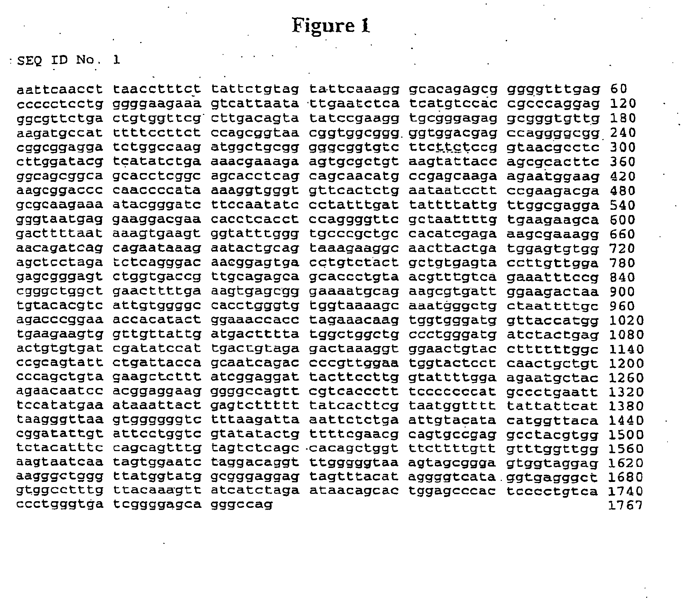

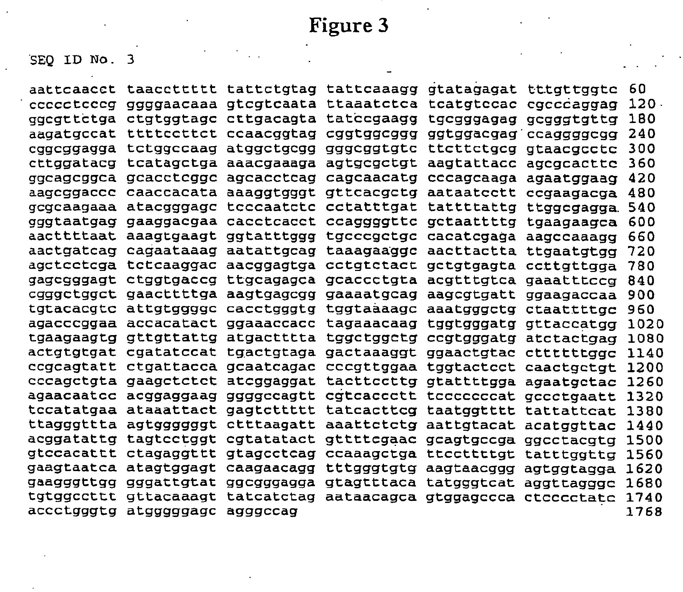

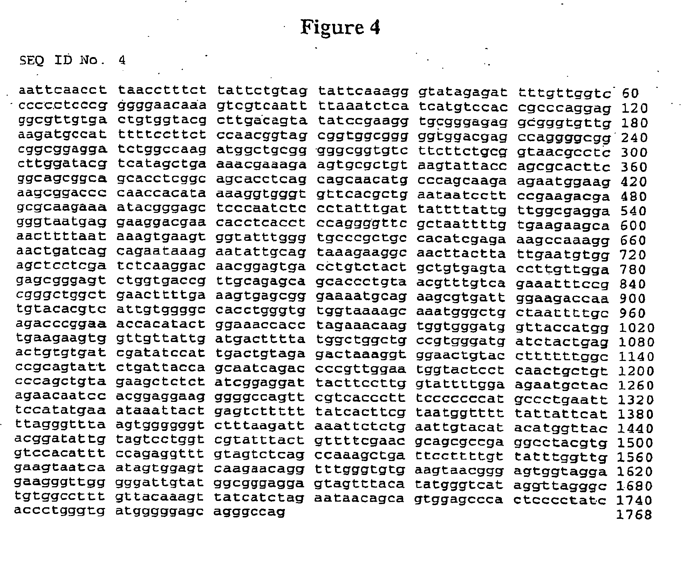

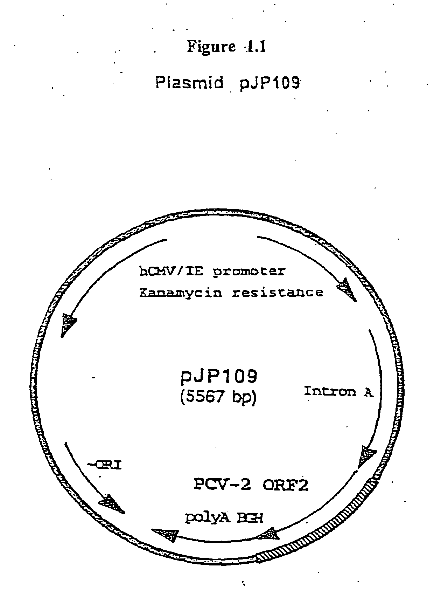

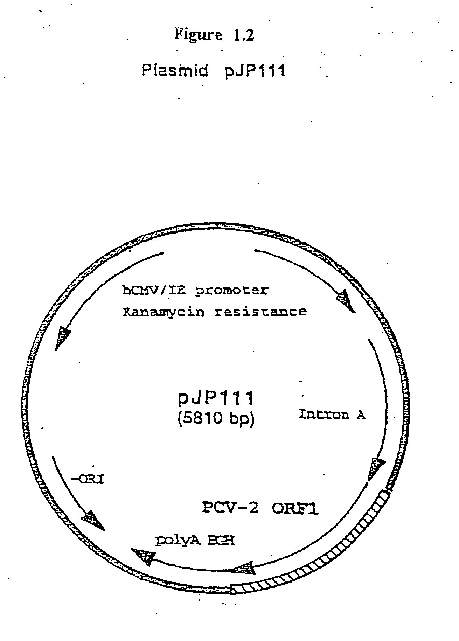

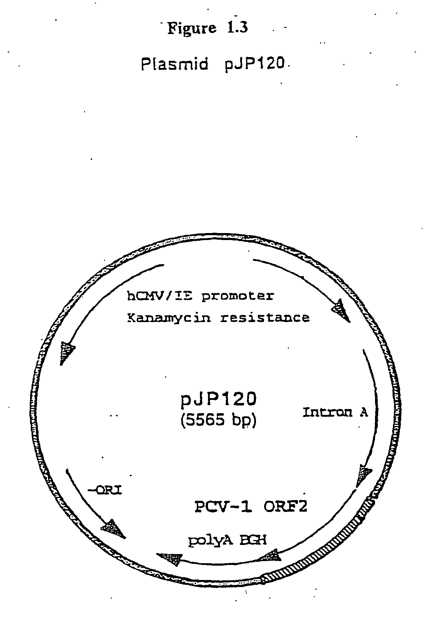

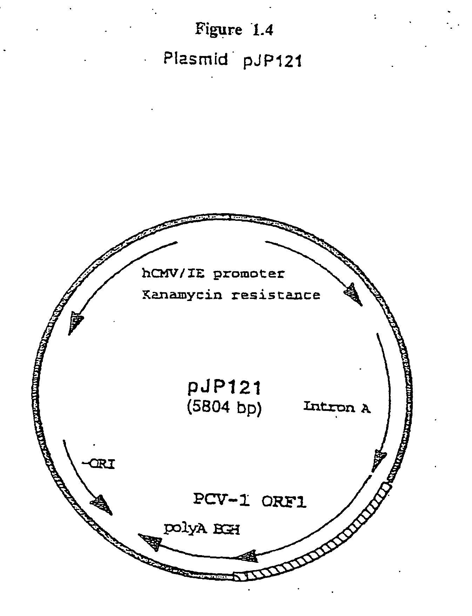

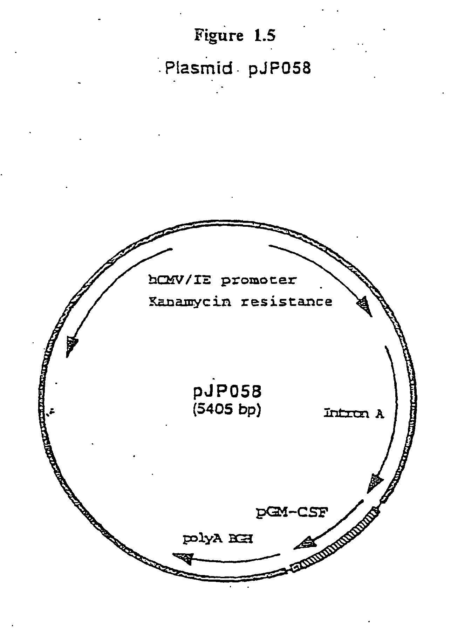

We describe that the immunogen in the immunogenic composition for use in the invention can be expressed from a DNA fragment containing a PCV-2 nucleotide sequence or fragment thereof (advantageously encoding at least one epitope), e.g. selected from the group consisting of the sequences designated by the references SEQ ID No: 1, SEQ ID No: 2, SEQ ID No: 3, SEQ ID No: 4, as well as SEQ ID No: 6 and SEQ ID No: 7, (Figures 1-4 and 6-7). The immunogen in the immunogenic composition can be expressed from a DNA fragment containing an ORF selected from ORFs 4 and/or 13, of a PCV-2 strain, in particular of one of the above identified strains (as designated in WO-A-99 18214-see also table 1 hereinafter). Thus, we describe that the immunogen or a portion thereof, such as an epitope of interest can be obtained by *in vitro* expression thereof from a recombinant or a vector. The immunogen may be further purified and/or concentrated by the conventional methods.

We describe that the immunogen in the immunogenic composition for use in the invention can be expressed *in vivo* by an expression vector comprising a DNA fragment containing a PCV-2 nucleotide sequence or fragment thereof (advantageously encoding at least one epitope), e.g. selected from the group consisting of the sequences designated by the references SEQ ID No: 1, SEQ ID No: 2, SEQ ID No: 3, SEQ ID No: 4, SEQ ID No: 6, as well as SEQ ID No: 6 and SEQ ID No: 7, (Figures 1-4 and 6-7). Similarly, the immunogen in the immunogenic composition can be expressed *in vivo* by an expression vector comprising a DNA fragment containing an ORF selected from ORFs 4 and/or 13, of a PCV-2 strain, in particular of one of the above identified strains. That is, we describe the immunogenic composition can comprise and expression vector that expresses the immunogen or a portion thereof, e.g., an epitope of interest, *in vivo.*

According to the invention the expression vector is a vector selected from DNA plasmids. We also describe the following vectors: bacteria such as *E. coli*, viruses such as baculovirus, herpesvirus such as Aujeszky's disease virus, adenovirus including porcine adenovirus, poxviruses, especially vaccinia virus, avipox virus, canarypox virus, and swinepox virus, *inter alia.* (The one skilled in the art can also refer to the U.S. applications of Audonnet et al. and Bublot et al., Serial Nos. 60/138,352 and 60/138,478, respectively, both filed June 10, 1999, in particular to the detailed description and more particularly to the examples (extracts of which are provided in "DNA VACCINE-PCV Example 12", and "PORCINE CIRCOVIRUS RECOMBINANT POXVIRUS VACCINE Example 13", respectively).

Accordingly, the invention comprehends DNA plasmid vectors containing nucleic acid molecules. We also describe expression products therefrom, compositions comprising such nucleic acid molecules and/or vectors and/or expression products, as well as methods for making and using any or all of these embodiments. We describe herein ORFs and/or fragments encoding an immunogen or epitope, as well as nucleic acid molecules of strains 1103 and/or 1121, in particular their ORFs 1 to 13, such as ORFs 4, 7, 10 and 13, preferably ORFs 4 and/or 13, and fragments thereof, as well as vectors comprising these nucleic acid molecules, compositions comprising these nucleic molecules, vectors, or expression products therefrom, compositions comprising such expression products, primers or probes for such nucleic acid molecules, and uses or methods involving these embodiments, e.g., for detecting, diagnosing, assaying for PCV-2, for inducing an immunogenic or protective response, and the like.

We also describe probes or primers from PCV-2 strains 1103 and/or 1121 which can be useful, for amplifying PCV-2 DNA, e.g., for preparing an expression vector. A probe or primer can be any stretch of at least 8, preferably at least 10, more preferably at least 12, 13, 14, or 15, such as at least 20, e.g., at least 23 or 25, for instance at least 27 or 30 nucleotides in PCV-2 genome or a PCV-2 gene which are unique to PCV-2, and possibly to these strains, or which are in PCV-2 and are least conserved among the PCV or circovirus family. As to PCR or hybridization primers or probes and optimal lengths therefor, reference is also made to Kajimura et al., GATA 7 (4): 71-79 (1990). Hybridization is advantageously under conditions of high stringency, as the term "high stringency" would be understood by those with skill in the art (see, for example,

Sambrook et al., 1989, Molecular Cloning: A Laboratory Manual, Cold Spring Harbor Laboratory Press, Cold Spring Harbor, N. Y. and Hames and Higgins, eds., 1985, Nucleic Acid Hybridization, IRL Press,. Oxford, U. K.). Hybridization will be understood to be accomplished using well-established techniques, including but not limited to Southern blot hybridization, Northern blot hybridization, *in situ* hybridization and, advantageously, Southern hybridization to PCR-amplified DNA fragments.

Like probes or primers, peptides which are not full-length PCV-2 proteins are described and can be any stretch of at least 8, preferably at least 10, more preferably at least 12, 13, 14, or 15, such as at least 20, e.g., at least 23 or 25, for instance at least 27 or 30 amino acids in PCV-2 which are unique to PCV-2, and possibly to these strains, or which are in PCV-2 and are least conserved among the PCV and/or circovirus family. Alternatively or additionally, the amino acids which are not full length PCV-2 proteins can be an epitopic region of a PCV-2 protein.

PCV-2 sequences are disclosed in Meehan et al., 1998 (Strain Imp. 1010; ORF1 nucleotides 398-1342; ORF2 nucleotides 1381-314; and correspond respectively to ORF4 and ORF13 in the invention terminology (see table 1), in U.S. application Serial No. 09/161,092 of 25 September 1998 and to COL4 and COL13 in WO-A-9918214). Several PCV-2 strains and their sequences are disclosed herein and called ImplO08, Imp999, ImplO11-48285, Imp1011-48121,1103 and 1121. Other strains are disclosed in A. L. Hamel et al. J. Virol. June 1998, vol 72, 6: 5262-5267 (GenBank AF027217) and in I. Morozov et al. J. Clinical Microb. Sept 1998 vol. 36, 9: 2535-2541, as well as GenBank AF086834, AF086835 and AF086836. We describe the use of these sequences, or ORFs therefrom, or regions thereof encoding an antigen or immunogen or epitope of interest.

The invention also encompasses the equivalent sequences to those used or mentioned herein and in documents cited herein; for instance, sequences that are capable of hybridizing to the nucleotide sequence under high stringency conditions (see, e.g., Sambrook et al. (1989).

Among the equivalent sequences, there may also be mentioned the gene fragments conserving the immunogenicity of the complete sequence, e.g., an epitope of interest.

The homology between the whole genome of PCV types 1 and 2 is about 75%. But within type 2, homology is generally above 95%. Thus, in the practice of the invention, use of any PCV-2 strain is encompassed by equivalence. A criteria can be that the strain is of type 2, e.g. that homology at the nucleotide level of the whole genome is equal or greater than 85%, advantageously 90% or greater, more advantageously 95% or greater, preferably 97, 98 or 99% or greater, with the strains disclosed herein, e.g. strain Imp1010.

Limits of the ORFs of strain Imp1010 are given in the following table 1:

| Name | Start | End | Strand | Size of the ORF (nucleotides (nt)) | Protein size (amino acids (aa)) |
|---|---|---|---|---|---|
| ORF1 | 103 | 210 | Sense | 108 nt | 35 aa |
| ORF2 | 1180 | 1317 | Sense | 138 nt | 45 aa |
| ORF3 | 1363 | 1524 | Sense | 162 nt | 53 aa |
| ORF4 | 398 | 1342 | Sense | 945 nt | 314 aa |
| ORF5 | 900 | 1079 | Sense | 180 nt | 59 aa |
| ORF6 | 1254 | 1334 | Sense | 81 nt | 26 aa |
| ORF7 | 1018 | 704 | Antisense | 315 nt | 104 aa |
| ORF8 | 439 | 311 | Antisense | 129 nt | 42 aa |
| ORF9 | 190 | 101 | Antisense | 90 nt | 29 aa |
| ORF10 | 912 | 733 | Antisense | 180 nt | 59 aa |
| ORF11 | 645 | 565 | Antisense | 81 nt | 26 aa |
| ORF12 | 1100 | 1035 | Antisense | 66 nt | 21 aa |
| ORF13 | 314 | 1381 | Antisense | 702 nt | 213 aa |

The ORFs are defined with respect to strain Imp1010. The invention also encompasses the use of the corresponding ORFs in any other PCV-2 strain, and any of the PCV-2 strains as defined herein or in documents cited herein. Thus, from the genomic nucleotide sequence, it is-routine art to determine the ORFs using a standard software, such as MacVector®. Also, alignment of genomes with that of strain 1010 and comparison with strain 1010 ORFs allows the one skilled in the art to readily determine the ORFs on the genome for another strain (e.g. those disclosed in WO-A-99 18214, say ImpI008, ImpI011-48121, ImpI011-48285, Imp999, as well as the new strains 1103 and 1121). Using software or making alignment is not undue experimentation and directly provides access to these ORFs.

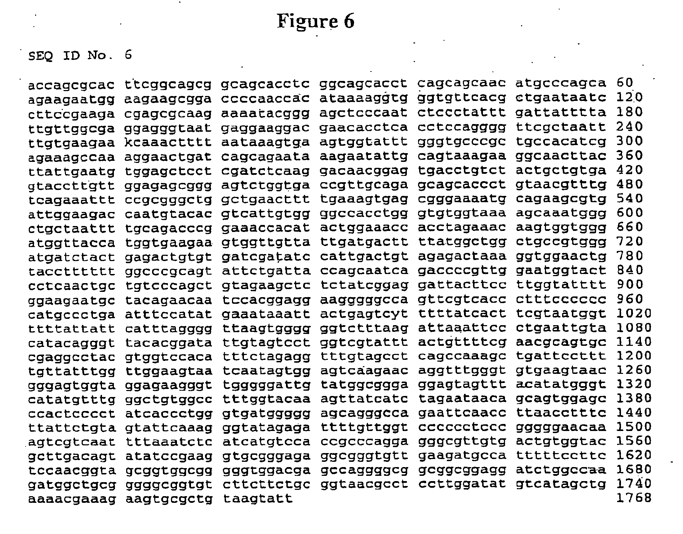

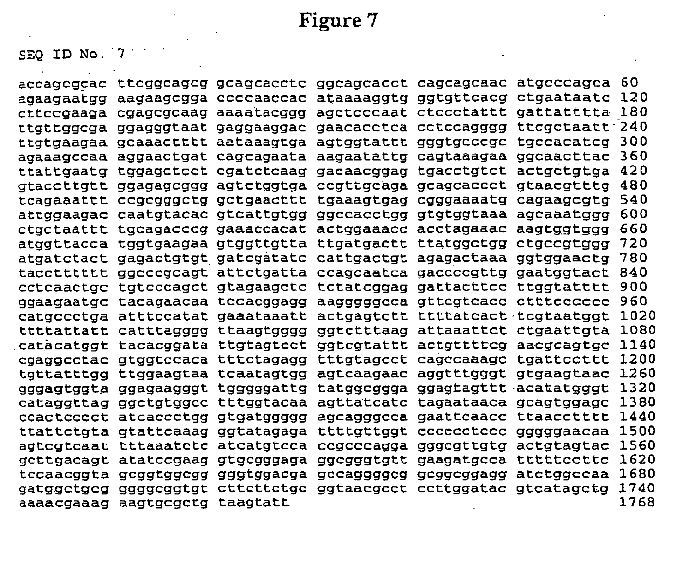

For example, referring to Figures 6 and 7, the corresponding ORFs of strains 1103 and 1121 are as given in the following Table 2:

| Name | Start | End | Strand | Size of the ORF (nucleotides (nt)) | Protein size (amino acids (aa)) |
|---|---|---|---|---|---|
| ORF1 | 1524 | 1631 | Sense | 108 nt | 35 aa |
| ORF2 | 833 | 970 | Sense | 138 nt | 45 aa |
| ORF3 | 1016 | 1177 | Sense | 162 nt | 53 aa |
| ORF4 | 51 | 995 | Sense | 945 nt. | 314 aa |
| ORF5 | 553 | 732 | Sense | 180 nt | 59 aa |
| ORF6 | 907 | 987 | Sense | 81 nt | 26 aa |
| ORF7 | 671 | 357 | Antisense | 315 nt | 104 aa |
| ORF8 | 92 | 1732 | Antisense | 129 nt | 42 aa |
| ORF9 | 1611 | 1522 | Antisense | 90 nt | 29 aa |
| ORF10 | 565 | 386 | Antisense | 180 nt | 59 aa |
| ORF11 | 298 | 218 | Antisense | 81 nt | 26 aa |
| ORF12 | 753 | 688 | Antisense | 66 nt | 21 aa |
| ORF13 | 1735 | 1037 | Antisense | 702 nt | 213 aa |

Also equivalent and useful in the practice of the invention are the nucleotide sequences which change neither the functionality nor the strain specificity (say of strain type 2) of the gene considered or those of the polypeptides encoded by this gene. The sequences differing through the degeneracy of the code are, of course, be included in the practice of the invention.

For ORF4, homology between PCV-1 and PCV-2 is about 86%, and for ORF13, the homology between PCV-1 and PCV-2 is about 66%. Thus, also equivalent sequences useful in the practice of the present invention, for ORF4, are those sequences having an homology of 95% or greater homology with ORF4 of strain Imp1010, and for ORF13, those sequences having an homology 95% or greater than ORF13 of strain Imp1010 (Using the terminology of table 1).

For homology regarding the other ORFs, which are described herein, one can determine those sequences which come from a PCV strain having an ORF4 and/or an ORF13 which have an homology as defined above with the corresponding ORF of strain 1010. For ORF7, we describe sequences having an homology that is advantageously equal to or greater than 80%, more advantageously 85% or greater, preferably 90% or 95% or greater with ORF7 of strain Imp1010. For ORF10, we describe sequences having an homology that is advantageously equal to or greater than 86%, more advantageously 90% or greater, preferably 95% or greater with ORF10 of strain Imp1010 (Using the terminology of table 1).

Also, equivalent sequences useful in the practice of this present invention, for ORF4 are those sequences having an homology of 95% or greater with ORF4 of strain Imp1010, and for ORF13, are those sequences having a homology of 95% or greater with ORF13 of strain Imp1010.

ORF4 and ORF13 has the potential to encode proteins with predicted molecular weights of 37.7 kD and 27.8 kD respectively. ORF7 and ORF10 (correspond to ORF3 and ORF4 in Meehan et al. 1998) has the potential to encode proteins with predicted molecular weights of 11.9 and 6.5 kD respectively. Sequences of these ORFs are also available in Genbank AF 055392. We describe that they can also be incorporated in plasmids and be used in accordance with the invention in combination with ORF4 and/or ORF13.

The other ORFs 1-3 and 5, 6, 8-9, 11-12 disclosed in the above table (COLs 1-3 and 5, 6, 8-9, 11-12 in WO-A-9918214), or region(s) thereof encoding an antigen or epitope of interest are described, e.g., alone or in combination or otherwise with each other or with the ORFs 4 and/or 13 or region(s) thereof encoding antigen(s) or epitope(s). Similarly, for homology, one can determine that there are equivalent sequences which come from a PCV strain having an ORF13 and/or an ORF4 which have an homology as defined above with the corresponding ORF of strain 1010 as defined herein or in Meehan et al., 1998. For ORF7, an equivalent sequence has homology thereto that is advantageously, for instance, equal or greater than 80%, for example 85% or greater, preferably 90% or 95% or greater with ORF7 of strain Imp1010. And, for ORF10, advantageously an equivalent sequence has homology that is equal or greater than 86%, advantageously 90% or greater, preferably than 95% or greater with ORF10 of strain Imp1010.

Nucleotide sequence homology can be determined using the "Align" program of Myers and Miller, ("Optimal Alignments in Linear Space", CABIOS 4, 11-17, 1988, and available at NCBI. Alternatively or additionally, the term "homology" or "identity", for instance, with respect to a nucleotide or amino acid sequence, can indicate a quantitative measure of homology between two sequences. The percent sequence homology can be calculated as (N_{ref} -N_{dif})*100/N_{ref}, wherein N_{dif} is the total number of non-identical residues in the two sequences when aligned and wherein N_{ref} is the number of residues in one of the sequences. Hence, the DNA sequence AGTCAGTC will have a sequence similarity of 75% with the sequence AATCAATC (N_{ref} = 8; N_{dif} = 2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur and Lipman, 1983 PNAS USA 80: 726, for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics^{™} Suite, Intelligenetics Inc. CA).. When RNA sequences are said to be similar, or have a degree of sequence identity or homology with DNA sequences, thymidine (T) in the DNA sequence is considered equal to uracil (U) in the RNA sequence.

RNA sequences within the scope of the invention can be derived from DNA sequences, by thymidine (T) in the DNA sequence being considered equal to uracil (U) in RNA sequences.

Additionally or alternatively, amino acid sequence similarity or identity or homology can be determined using the BlastP program (Altschul et al., Nucl. Acids Res. 25,3389-3402) and available at NCBI. The following references provide algorithms for comparing the relative identity or homology of amino acid residues of two proteins, and additionally or alternatively with respect to the foregoing, the teachings in these references can be used for determining percent homology or identity: Needleman SB and Wunsch CD, "A general method applicable to the search for similarities in the amino acid sequences of two proteins," J. Mol. Biol. 48:444-453 (1970); Smith TF and Waterman MS, "Comparison of Bio-sequences, " Advances in Applied Mathematics 2:482-489 (1981); Smith TF, Waterman MS and Sadler JR, "Statistical characterization of nucleic acid sequence functional domains," Nucleic Acids Res., 11:2205-2220 (1983); Feng DF and Dolittle RF, "Progressive sequence alignment as a prerequisite to correct phylogenetic trees, " J. of Molec. Evol., 25:351-360 (1987); Higgins DG and Sharp PM, "Fast and sensitive multiple sequence alignment on a microcomputer CABIOS, 5: 151-153 (1989); Thompson JD, Higgins DG and Gibson TJ, "ClusterW: improving the sensitivity of progressive multiple sequence alignment through sequence weighing, positions-specific gap penalties and weight matrix choice, Nucleic Acid Res., 22:4673-480 (1994); and, Devereux J, Haeberlie P and Smithies O, " A comprehensive set of sequence analysis program for the VAX, " Nucl. Acids Res., 12: 387-395 (1984).

We further describe uses of a PCV-2 immunogen, either alone or in further combination with an immunogen of another porcine pathogen to generate compositions according to the invention, e.g., admixing the ingredients; and, the invention also therefore comprehends kits wherein components are individually contained and optionally the containers are packaged together for admixture and/or administration, wherein the kit can also optionally include instructions for admixture and/or administration.

While the invention has been discussed in terms of administering to female pigs immunogenic compositions comprising a PCV-2 immunogen, the invention can also comprehend administering such compositions to a sow or gilt and/or to boar as described herein. Thus, both mother and offspring (e.g., sow, gilt) and boar can be administered compositions of the invention. Accordingly, populations of pigs can be administered compositions for use in the invention.

We describe that immunogenic and vaccine compositions may comprise immunogens from more than one PCV-2 strain. For example, it is possible to combine immunogens from strains 1121 and 1103, from one or both of these strains with at least one other strain disclosed herein, or any other combination.

We describe methods allowing the one skilled in the art to evaluate the efficacy of vaccines against PCV-2. A first method is an ELISA method or with seroneutralization. A second method is a vaccination followed by challenge with a virulent PCV-2 strain, e.g. one of the strains disclosed herein. In other words, the invention allows one to check for PCV immunogens, including PCV-1. immunogens, able to elicit an immunogenic or protective response against PCV-2. Such PCV immunogens are then useful to prevent or treat pigs against in particular myocarditis and/or abortion and/or intrauterine associated with PCV-2, as well as against PMWS and/or other pathologic sequelae associated therewith.

We provide immunogenic or vaccinal compositions comprising a PCV immunogen and able to elicit an immunogenic or protective response against PCV-2. The invention relates also to methods of immunization or vaccination using such an immunogen, as well as to the use of such an immunogen to produce such an immunogenic or vaccinal composition.

The invention shall be further described by way of the following Reference Examples, Example And Results, provided for illustration and not to be considered a limitation of the invention.
Figure 1 depicts SEQ ID No. 1, the PCV DNA sequence of the genome of the Imp. 1011-48121 strain.
Figure 2 depicts SEQ ID No. 2, the DNA sequence of the genome of the Imp. 1011-48285 strain.
Figure 3 depicts SEQ ID No. 3, the DNA sequence of the genome of the Imp. 999 strain.
Figure 4 depicts SEQ ID No. 4, the DNA sequence of the genome of the Imp. 1010 strain.

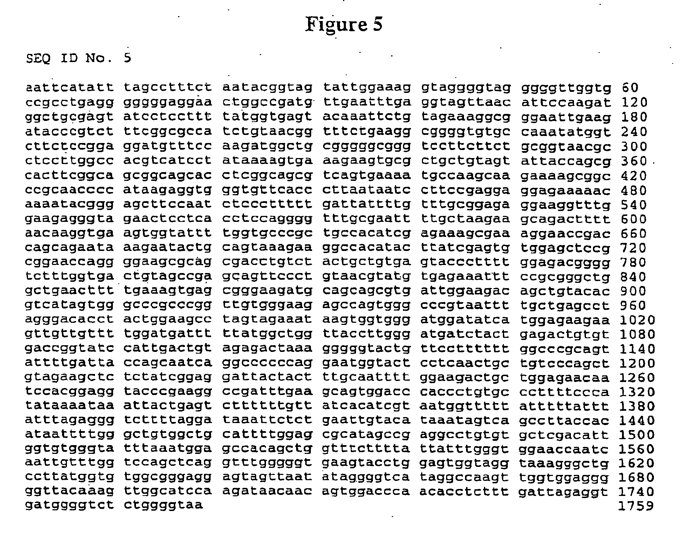

Figure 5 depicts SEQ ID No. 5, the reference DNA sequence of the genome of the PK/15 strain.
Figure 6 depicts SEQ ID No. 6, the DNA sequence of the genome of the 1103 strain, isolated in Alberta, Canada. k means g (G) or t (T), y means c (C) or t (T). These variations of sequence were observed in the viral population.
Figure 7 depicts SEQ ID No. 7, the DNA sequence of the genome of the 1121 strain, isolated in Saskatoon, Canada.
Figures 1.1 to 1.5 are described in Example 12.

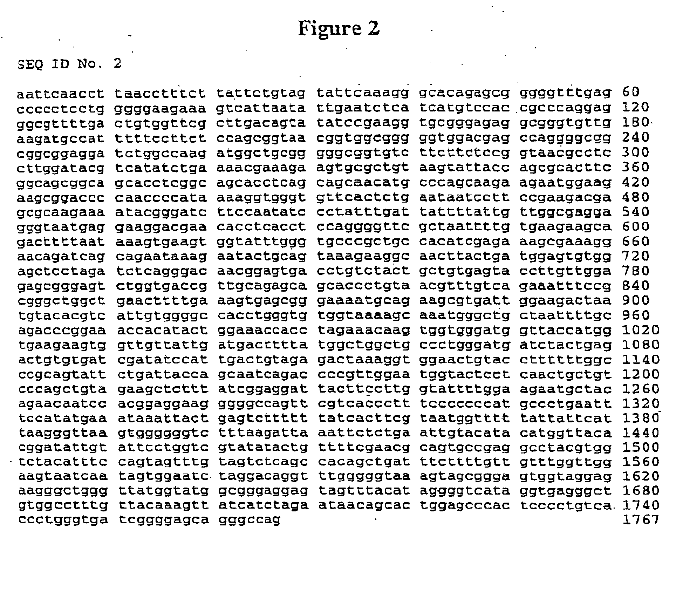

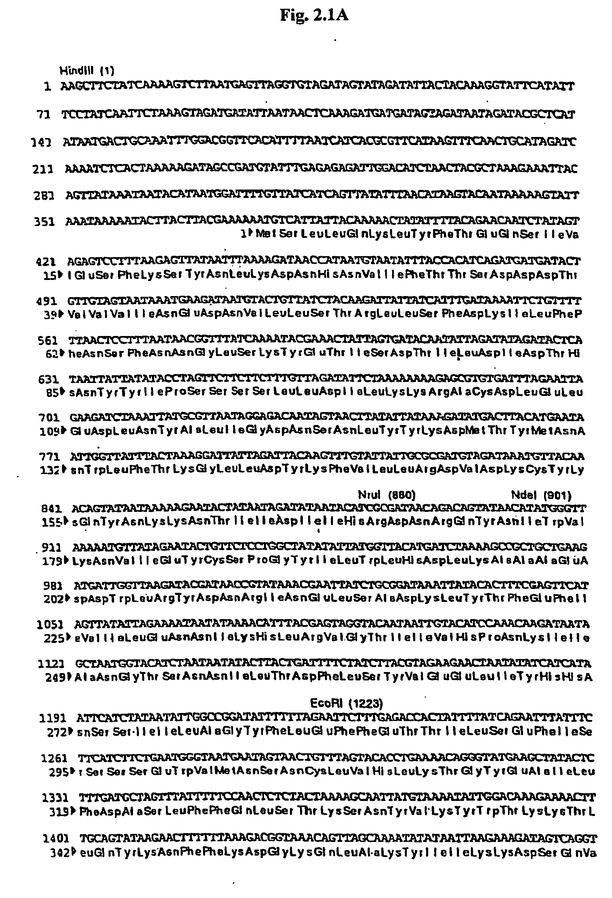

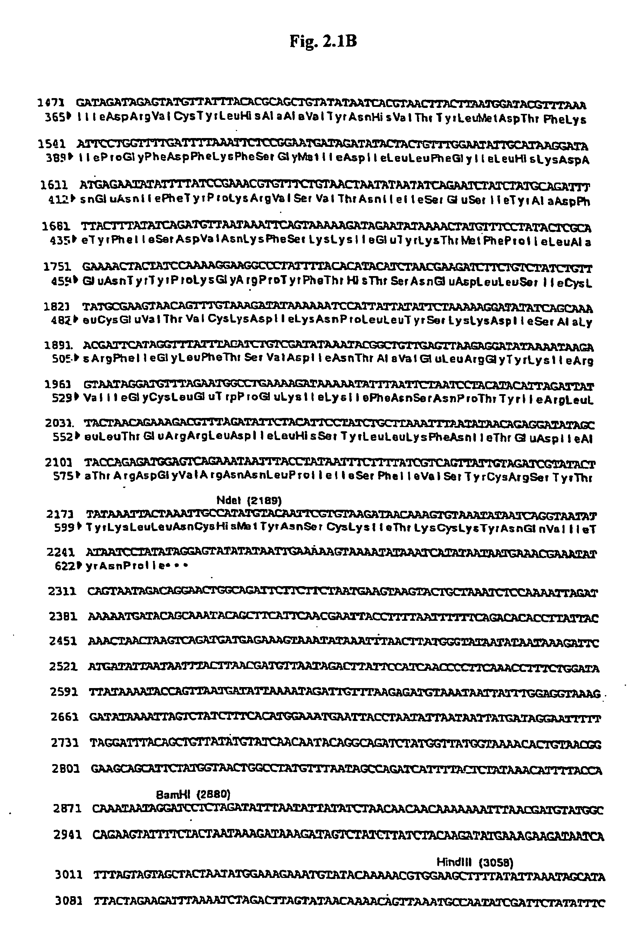

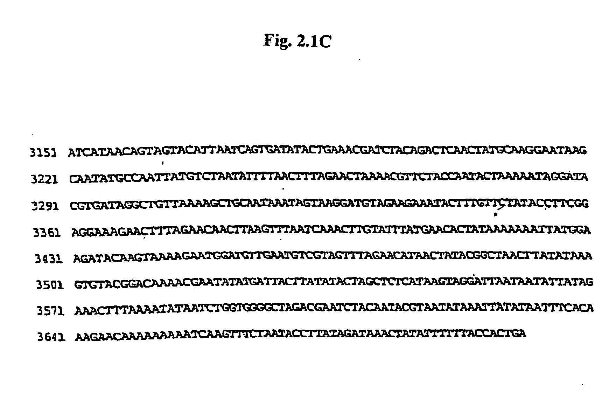

Figures 2.1 to 2.6 are described in Reference Example 13.

### EXAMPLE AND RESULTS

### REFERENCE EXAMPLE/RESULT 1: MYOCARDITIS, ABORTION AND INTRAUTERINE INFECTION ASSOCIATED WITH PCV-2

Late term abortions and farrowings with both stillborn and mummified piglets occurred in a new 450-female pig swine facility as it was brought into production.

Pseudopregnancy was also observed in several gilts. Gilts received two doses of an inactivated vaccine containing parvovirus and leptospiral immunogens prior to breeding.

A litter received for postmortem examination consisted of nine fetuses that appeared to have died at various stages of gestation. There were 2 mummified, 2 macerated, 3 autolyse and 2 fresh, stillborn piglets. Lesions were observed on gross pathological examination in one partially autolysed fetus only. In this fetus both ventricles of the heart were dilated, the liver was enlarged and firm and there was both hydrothorax and ascites. Histopathologically, there were extensive areas of myocardial degeneration or necrosis with edema and mild fibrosis, and a diffuse moderate -infiltration of lymphocytes and macrophages. There was marked generalized hepatic congestion and hepatocellular loss. The spleen and kidneys were also congested. Significant histological lesions were not detected in the other fetuses.

Immunohistochemical staining for PCV-2 was performed as previously described using a rabbit polyclonal antiserum and a monoclonal antibody that were raised against PCV- 2. On sections of formalin-fixed, routinely processed and embedded tissue (Ellis et al., 1998; Ellis et al., 1999). In the fetus with dilated cardiomyopathy there was extensive staining for PCV-2 antigen throughout the affected myocardium. Staining was most extensive in areas of necrosis and appeared to involve primarily myocytes. Both cytoplasmic and nuclear staining was present. In multiple fetuses there was extensive staining in the liver. In some sections it appeared to involve primarily sinusoidal endothelium and Kupfer cells, while in other fetuses, including the one with myocarditis, there was also nuclear and cytoplasmic staining of hepatocytes. Positively stained cells were scattered throughout the lung, and multifocally in the kidney. Polymerase chain reaction for PCV-2 was performed as previously described using frozen tissue (Ellis et al., 1999). PCR product of the expected size for PCV-2 was amplified from fetal tissue. PCV-2 was isolated from the fetus with myocarditis and a pool of tissues from other fetuses in the litter by inoculating tissue homogenates onto PCV-free PK-15 cells.

Fetal tissues were also examined for other viral pathogens that have been associated with fetal injury and abortions in swine, including, porcine parvovirus (PPV), porcine reproductive and respiratorysyndrome virus (PRRSV), encephalomyocarditis (EMCV), and enteroviruses. PPV antigen was not detected by fluorescent antibody testing (FAT) on frozen sections of lung, liver, and spleen from the mummified or stillborn fetuses. Homogenates of liver, lung, and spleen from the aborted fetuses were also inoculated into cultures of PCV-free PK-15 cells, primary porcine fallopian tube cells and Vero cells. Cytopathic viruses were not detected after three passages. Tissues were negative for PPV using PCR. PRRSV antigen was not detected by immunohistochemical staining.

Thus, there were fetal lesions and abortion directly associated with PCV-2. These results also show vertical transmission of the virus.

In a previous study, PCV-1 was isolated from 2 of 160 pig fetuses examined, implying that this group of viruses can be vertically transmitted; however, PCV-1 antigen could not be associated with any lesions in the tissue (Allan et al., 1995). The exclusion of other agents that have been associated with fetal lesions and abortion in swine, including, PPV (Bolt et al., 1997; Molitor et al., 1991), PRRSV (Lager et al., 1996), EMCV (Kim et al., 1989), and enterovirus (Molitor et al., 1991) indicate that PCV-2 can cause significant fetal pathology and subsequent abortion.

The wasting syndrome associated with PCV-2 infection most often occurs in 5-12 week old pigs (Allan et al., 1998; Ellis et al., 1998). Experimental infection of neonatal swine indicates a relatively long prodromal period between infection and the development of clinical signs associated with PCV-2 (Allan et al. 1999; Ellis et al. 1999). The findings herein show that the virus is transmitted vertically or in the perinatal period. Not only may interuterine vertical transmission of PCV-2 result in abortion, but it is possible that sublethally *in utero*- infected piglets may be the animals that subsequently develop PMWS.

Furthermore, these results show that inoculation of female pigs with a composition comprising an PCV-2 immunogen (which composition can also include an immunogen from another porcine pathogen, e.g., porcine parvovirus), prior to breeding or serving, or prior to the perinatal period and/or during gestation can prevent myocarditis and/or abortion and/or intrauterine infection associated with porcine circovirus-2, as well as post-weaning multisystemic wasting syndrome and other pathologic sequelae associated with PCV-2, by eliciting an immunological response or antibodies against PCV-2.

We describe that of course, compositions, methods, and other aspects of the invention can be used or practiced in animals other than pigs, e.g., sheep, bison, cattle, wild boar, for instance, if PCV-2 infects such other animals.

### REFERENCE EXAMPLE/RESULT 2: MYOCARDITIS, ABORTION AND INTRAUTERINE INFECTION ASSOCIATED WITH PCV-2

The presence of PCV-2 in neonatal piglets suggests that vertical transmission may be an important means of viral transmission. This mode of transmission may be related not only to reproductive failure, but also to the development of multisystemic disease later in life. It is of interest to determine whether previously undetected PCV-2 (and PCV-I) has been vertically transmitted in pork producing areas where PMWS, and by extension PCV-2 infection, has been endemic for at least several years.

Thirty eight submissions involving reproductive failure received in the diagnostic laboratory at the Western College of Veterinary Medicine (WCVM), University of Saskatchewan, Saskatoon, Canada, over a four-year period from a total of 30 high health herds in Canada were evaluated. Five of the farms from which the samples were obtained had diagnose cases of PMWS. Twenty-seven of the thirty-eight submissions (71 %) were classified as abortions; five of these (13%) also involved at least one mummified fetus. Of the remaining 10 cases: 5 involved stillborn piglets along, with nonviable piglets (13%); 2 with stillborn and one or more mummified feti (5%); 2 with only stillborn piglets (5%); and one with only mummified feti (2.5%). Routine diagnostics for pathogens other than circovirus revealed 4 cases (11%) in which the etiology was determined to be porcine parvovirus and 2 cases (5%) in which the etiology was determined to be of bacterial origin. Gross necropsies were performed and tissues were collected and fixed in buffered formalin (fixation time 24-72 hrs) and, in most cases, fresh tissues were also submitted for routine microbiological evaluation. None of these cases had been previously tested for PCV-2.

The PCR technique used for the detection of PCV-1 and PCV-2 was performed as previously described (Tischer et al. 1974). PCV-1 was not detected by PCR in any submissions comprising reproductive failure from the four-year period PCV-2 was detected by PCR in two different submissions that originated from the same multi-site pork production unit on two separate occasions in the spring of the last year in the four-year period. The first of these submissions comprised a litter of piglets with gross evidence of myocarditis, cardiac hypertrophy, and chronic passive congestion.

Immunohistochemical identification, of PCV-2 in tissues was performed as previously described (Tischer et al. 1974). Immunohistochemical staining (1HC) for PCV-2 was positive in hearts from all six of the piglets that were submitted, while 4 of 6 were positive by PCV-2 PCR (see following Table).

**Table: Detection of PCV-2 in the formalin fixed hearts of porcine with myocarditis by PCR, IHC and viral isolation in cell culture.**

| PCV-2 postive tissues | PCR | IHC | Virus Isolation |
|---|---|---|---|
| Fixed | 5/6 | 6/6 | N/A |
| Frozen | 4/4 | N/A | 2/4 |

The second submission from the same farm consisted of a litter of four piglets in which 2 were stillborn and 2 others died shortly after birth. All four piglets also had gross evidence of a severe, difuse myocarditis, cardiac hypertrophy, and chronic passive congestion. Only fresh frozen heart, and pooled lung/spleen tissues were submitted for analysis. PCV-2 PCR was positive in the hearts of 2 of 4 piglets and in the pooled lung and splenic tissues of 4 of 4 piglets. Isolation of PCV-2 from affected hearts and/or pooled lung and splenic tissue was positive in 2 of the 4 cases that were PCV-2 positive by PCR. Based on serology and/or PCR, other agents associated with reproductive failure in swine, including porcine reproductive and respiratory syndrome virus and porcine parvovirus were apparently circulating in the breeding herd. However, these agents could not be shown to be associated with the severe cardiac (or other) lesions in the affected piglets; but, they may contribute to PMWS.

PCV-2 was not detected by PCR or IHC in any representative cases of reproductive failure submitted during the first three years of the four-year period (it was detected in cases of reproductive failure submitted during the last year of the four-year period). In order to rule out damage to DNA due to formalin fixation as a possible adverse factor limiting the ability to detect PCV-2 by PCR, PCR was performed on tissues collected from four weanling piglets with PMWS, PCV-2 DNA was amplified in all fixed tissues tested, including; lung, liver, kidney and bronchial lymph node, from all four individuals. Moreover, the sensitivity of the PCR PCV-2 was independent of the length of time that each tissue was fixed in formalin.

These results confirm and extend the previous observation (West et al. 1999) that PCV-2 can be vertically transmitted and can be present in large amounts within lesions from piglets infected *in utero*. Vertical transmission of PCV-2 virus and resultant fetal damage, such as myocarditis, is an additional disease manifestation of PCV-2. Furthermore, the failure to detect PCV-2 in cases of reproductive failure prior to the last year of the four-year period from an endemic area of PCV-2 infection may indicate that vertical transmission was not the primary mechanism responsible for the initial dissemination of viral infection. Sexual, as well as vertical, modes of transmission can be attributed to the spread of PCV-2 infection in pigs.

### REFERENCE EXAMPLE/RESULT 3: CULTURE AND ISOLATION OF THE PORCINE CIRCOVIRUS STRAINS

Viruses 1103 and 1021 were isolated respectively in Alberta, respectively Saskatoon, Canada, from abortive cases according to the method described in J. Ellis et al. Can. J. Vet, 1998, vol 39, 44-51.

Viral culture is carried out on PK/15 cell cultures, known to be uncontaminated with the porcine circovirus (PCV), pestiviruses, porcine adenoviruses and porcine parvovimses (Allan G. et al Pathogenesis of porcine circovirus experimental infections of colostrum- deprived piglets and examination of pig foetal material. Vet. Microbiol. 1995,44,49-64).

Monolayers of PK/15 cells are dissociated by trypsinization (with a trypsin-versene mixture) from confluent cultures, and taken up in MEM-SA medium containing 15% foetal calf serum not contaminated by pestivirus (= MEM-G medium) in a final concentration of about 400,000 cells per ml. 10 ml aliquot fractions of this cell suspension are then mixed with 2 ml aliquot fractions of the inocula described above, and the final mixtures is aliquoted in 6 ml volumes in two Falcon flasks of 25 cm². These cultures are then incubated at +37°C for 18 hours under an atmosphere containing 10% CO₂.

After incubation, the culture medium of the semi-confluent monolayers were treated with 300 mM D-glucosamine (Cat # G48175, Sigma-Aldrich Company Limited, Poole, UK) (Tischr I. et al., Arch. Virol., 1987 96 39-57), then incubation was continued for an additional period of 48-72 hours at +37°C. Following this last incubation, one of the two Falcons of each inoculum was subjected to 3 successive freeze/thaw cycles. The PK/15 cells of the remaining Falcon were treated with a trypsin-versene solution, resuspended in 20 ml of MEM-G medium, and then inoculated into 75 cm² Falcons at a concentration of 400,000 cells/ml. The freshly inoculated flasks were then "superinfected" by addition of 5 ml of the corresponding lysate obtained after the freeze/thaw cycles.

### REFERENCE EXAMPLE/RESULT 4: TECHNIQUE FOR THE DETECTION OF PCV. BY IMMUNOFLUORESCENCE

The initial screening of all the cell culture preparations fixed with acetone was carried out by an indirect immunofluorescence technique (IIF) using a 1/100 dilution of a pool of adult pig sera. This pool of sera comprises sera from 25 adult sows from Northern Ireland and is known to contain antibodies against a wide variety of porcine viruses, including PCV: porcine parvovirus, porcine adenovirus, and PRRS virus. The IF technique was carried out by bringing the serum (diluted in PBS) into contact with the cell cultures for one hour at +37°C, followed by two washes in PBS. The cell cultures were then stained with a 1/80 dilution in PBS of a rabbit anti-pig immunoglobulin antibody conjugated with fluorescein isothiocyanate for one hour, and then washed with PBS and mounted in glycerol buffer prior to the microscopic observation under ultraviolet light.

### REFERENCE EXAMPLE/RESULT 5: PRODUCTION OF PCV ANTIGENS BY IN VITRO CULTURE

The culture of the noncontaminated PK/15 cells and the viral multiplication were carried out according to the same methods as Reference Example 1. The infected cells are harvested after trypsinization after 4 days of incubation at 37°C and enumerated. The next passage is inoculated with 400,000 infected cells per ml.

The various PCV-2 strains disclosed herein, e.g. strains 1103 and 1121 are so cultivated.

### REFERENCE EXAMPLE/RESULT 6: TITRATION OF PCV-2

Titration is carried out in 96-well microplates. A suspension of PK/15 cells (150 000 cells per ml) is first introduced (100 µl per well). Then dilutions of the viral culture are done and 100 µl thereof are introduced in the wells. Incubation is done at 37°C with CO₂. After 24h, there is carried out a treatment with glucosamine half an hour at 37°C (for the conditions see example 3). The culture medium is then removed and fresh medium is introduced.

Incubation is conducted 72h at 37°C. Revelation of the foci is done using an anti-PCV-2 monoclonal antibody and a FITC labelled mouse conjugate.

This method can be used to titration for preparing inactivated as well as live attenuated PCV-2.

### REFERENCE EXAMPLE/RESULT 7: INACTIVATION OF THE VIRAL ANTIGENS

At the end of the viral culture, the infected cells are harvested and lysed using ultrasound (Branson Sonifier) or with the aid of a rotor-stator type colloid mill (UltraTurrax, IKA). The suspension is then centrifuged at 3700 g for 30 minutes. The viral suspension is inactivated with 0.1% ethyleneimine for 18 hours at +37°C or with 0.5% beta-propiolactone for 24 hours at +28°C. If the virus titre before inactivation is inadequate, the viral suspension is concentrated by ultrafiltration using a membrane with a 300 kDa cut-off (Millipore PTMK300). The inactivated viral suspension is stored at +5°C.

### REFERENCE EXAMPLE/RESULT 8: PREPARATION OF THE VACCINE IN THE FORM OF AN EMULSION BASED ON MINERAL OIL

The vaccine is prepared according to the following formula:
- suspension of inactivated porcine circovirus: 250 ml
- Montanide® ISA 70 (SEPPIC): 750 ml
The aqueous phase and the oily phase are sterilized separately by filtration. The emulsion is prepared by mixing and homogenizing the ingredients with the aid of a Silverson turbine emulsifier.

One vaccine dose contains about 10^{7.5} TCID50. The volume of one vaccine dose is 0.5 ml for administration by the intradermal route, and 2 ml for administration by the intramuscular route.

This vaccine is used in a vaccination programme against the multisystemic wasting syndrome in combination with the Parvovax^{®} vaccine.

### REFERENCE EXAMPLE/RESULT 9: PREPARATION OF THE VACCINE IN THE FORM OF A METABOLIZABLE OIL-BASED EMULSION

The vaccine is prepared according to the following formula:
- suspension of inactivated porcine circovirus 200 ml
- Dehymuls HRE 7 (Henkel): 60 ml
- Radia 7204 (Oleofina): 740 ml

The aqueous phase and the oily phase are sterilized separately by filtration. The emulsion is prepared by mixing and homogenizing the ingredients with the aid of a Silverson turbine emulsifier.

One vaccine dose contains about 10^{7.5} TCID50. The volume of one vaccine dose is 2 ml for administration by the intramuscular route.

This vaccine is used in a vaccination programme against the multisystemic wasting syndrome in combination with the Parvovax^{®} vaccine.

### EXAMPLE/RESULT 10: VACCINATION OF PIGLETS WITH DNA (PLASMID) VECTOR (according to the invention)

Groups of 3 or 4 piglets, caesarian-derived day 0 are placed into isolators. The piglets are vaccinated day 2 either with (A) a plasmid comprising ORF 13 or with (B) a mixture of this plasmid and another plasmid comprising ORF 4, and with a physiological solution for the control group. Each plasmid is diluted in sterile physiological solution (NaCl 0,9%) at 250 µg/µl final concentration. A 2 ml volume is injected by intramuscular route in two points of 1 ml (1 point each side of the neck). A second injection of vaccine or placebo is administered day 14. Vaccination with DNA is well tolerated by piglets and no evidence for adverse reaction to vaccination is noted. The piglets are challenged day 21 by oronasal administration of PCV-2 viral suspension, 1 ml in each nostril. After challenge piglets are weighed once a week. Rectal temperatures are recorded on days 17, 21, 22, 24, 27, 29, 31, 34, 37, 41, 44. Day 44 fecal swabs are collected from each piglet for PCV-2 shedding. The virus is detected and quantified by quantitative PCR. Day 45 necropsies are performed and tissue samples are collected for virus isolation.
• Clinical symptoms:
There is no significant difference for the mean body weight gains or the mean body temperatures between groups.

• Necropsy lesions :
The only gross finding noted in pigs at termination is bronchial lymphadenopathy. The lesions are scored according the following criteria.
0 = no visible enlargement of lymph nodes
1 = mild lymph nodes enlargement, restricted to bronchial lymph nodes
2 = moderate lymph nodes enlargement, restricted to bronchial lymph nodes
3 = severe lymph nodes enlargement, extended to bronchial submandibullar prescapsular and inguinal lymph nodes.

std is an abbreviation for standard deviation

| **Groups** | **Lymphadenopathy scores** | | |
|---|---|---|---|
| | **mean** | **std** | **N** |
| | | | |
| (A) | 1.2 | 1.3 | 4 |
| (B) | 2.0 | 1.7 | 3 |
| controls | 3.0 | 0.0 | 3 |

| | | | |
|---|---|---|---|
| N = number of piglets in each group | | | |

A reduction of the lymph node lesions is observed in 3 out 4 piglets immunized with (A) and 1 out 3 piglets immunized with (B) mixture. This difference is not significant (p>0.05) due to the high value of the standard deviations (std).
- Virus load in lymph nodes tissues:
   Quantitative virus re-isolation is performed on tissue homogenates prepared from bronchial and mesenteric lymph nodes.

The data presented correspond to the virus titers in tissue homogenates after transformation in log₁₀.

| **PCV-2 titers** | | | | | |
|---|---|---|---|---|---|
| **Groups** | **Bronchial LN** | | **Mesenteric LN** | | |
| | mean | std | mean | std | N |
| | | | | | |
| (A) | 0.9 | 0.8 | 0.9 | 0.8 | 4 |
| (B) | 0.7 | 0.6 | 0.2 | 0.2 | 3 |
| Controls | 2.0 | 1.1 | 1.8 | 1.1 | 4 |

Bronchial lymph nodes seem to contain the most infectious virus. A reduction of the viral load is observed in bronchial and mesenteric lymph nodes from piglets immunized with either (A) or (B) mixture. This reduction is significant (p ≤ 0.05 for the plasmids mixture.
- Viral excretion:
   Post challenge fecal swabs are assessed for schedding PCV-2 by PCR based on amplification of PCV-2 ORF 13. Each assay is performed in triplicate on 2 ml of sample.
   Unvaccinated controls are negative for PCV-2 prior challenge and positive after challenge confirming the validity of the PCR assay.

Value are expressed as log₁₀ (number of molecules of PCV-2 DNA in 2 µl sample).

| **Log₁₀ number of PCV-2 DNA molecules** | | | |
|---|---|---|---|
| ***Groups*** | ***mean*** | ***std*** | ***N*** |
| | | | |
| (A) | 3.3 | 0.3 | 4 |
| (A) | 2.9 | 0.7 | 3 |
| Controls | 3.6 | 0.6 | 4 |

The differences between groups are not significant (p > 0.05).

### REFERENCE EXAMPLE/RESULT 11: VACCINATION OF PIGLETS WITH CANARYPOX LIVE VECTOR AND RESULTS

Groups of 3 or 4 piglets, caesarian-derived day 0 are placed into isolators. Day 2 the piglets are vaccinated with 10⁸ pfu of (C) a canarypox comprising ORF 13, or (D) of a canarypox comprising ORF 13 and ORF 4, or parental canarypox, in 1 ml of PBS, by intramuscular route on the side of the neck. A second injection of vaccine or placebo is administered at day 14. Vaccination with canarypox is well tolerated by piglets and no evidence for adverse reaction to vaccination is noted. The piglets are challenged day 21 by oronasal administration of a PCV-2 viral suspension, 1 ml in each nostril. Day 45 necropsies are performed and samples of tissues are collected for virus isolation.

### Necropsy results :

• PMWS is characterized generally by lymphadenopathy and more rarely by hepatitis or nephritis. So the gross findings in lymph nodes are scored for each piglet in the following manner : 0 = no visible enlargement of lymph nodes ; 1 = mild lymph nodes enlargement, restricted to bronchial lymph nodes ; 2 = moderate lymph nodes enlargement, restricted to bronchial lymph nodes ; 3 = severe lymph nodes enlargement, extended to bronchial, submandibullar prescapular and inguinal lymph nodes.

| **Groups** | **Scores** |
|---|---|
| (C) | 0.5 |
| | 0.0 |
| | 0.0 |
| | 1.0 |
| mean | 0.38 |
| standard deviation | 0.48 |
| | |
| (D) | 0.0 |
| | 0.5 |
| | 0.5 |
| | 1.0 |
| mean | 0.5 |
| standard deviation | 0.41 |
| | |
| Controls | 2.0 |
| | 2.5 |
| | 2.5 |
| | 2.5 |
| mean | 2.38 |
| standard deviation | 0.25 |

Bronchial lymphadenopathy for PCV-2 is a prominent gross finding. A significant reduction of the lymph nodes lesion in relation to control group is observed after immunization with (C) and (D) (p ≤ 0.05).

### DNA Vaccine-PCV EXAMPLE 12

The following passages are extracts from US provisional application 60/138,352.

The PCV-2 sequences used in these examples are derived from Meehan et al. supra (ORF1 nucleotides 398-1342; ORF2 nucleotides 1381-314; and correspond respectively to ORF4 and ORF13 in US 09/161,092 of 25 September 1998 and to COL4 and COL13 in WO-A-9918214).

The word plasmid is here intended to cover any DNA transcription unit in the form of a polynucleotide sequence comprising the PCV sequence to be expressed and the elements necessary for its expression *in vivo*. The circular plasmid form, supercoiled or otherwise, is preferred. The linear form is also included within the scope of the invention.

The Figures show the plasmids called pJP109 (containing the ORF2 gene of PCV-2, Figure 1.1), pJP111 (containing the ORF1 gene of PCV-2, Figure 1.2), and reference plasmid pJP120 (containing the ORF2 gene of PCV-1, Figure 1.3) and reference plasmid pJP121 (containing the ORF1 gene of PCV-1, Figure 1.4).

Each plasmid comprises a promoter capable of ensuring, in the host cells, the expression of the inserted gene under its control. It is in general a strong eukaryotic promoter and in particular a cytomegalovirus early promoter CMV-IE, of human or murine origin, or optionally of other origin such as rat or guinea pig. More generally, the promoter is either of viral origin or of cellular origin. As a viral promoter other than CMV-IE, there may be mentioned the SV40 virus early or late promoter or the Rous Sarcoma virus LTR promoter. It may also be a promoter from the virus from which the gene is derived, for example the promoter specific to the gene. As cellular promoter, there may be mention, the promoter of a cytoskeleton gene, such as for example the desmin promoter, or alternatively the actin promoter. When several genes are present in the same plasmid, they may be provided in the same transcription unit or in two different units.

The plasmids may also comprise other transcription regulating elements such as, for example, stabilizing sequences of the intron type, preferably intron II of the rabbit β-globin gene (van Ooyen et al. Science, 1979,206: 337-344), signal sequence of the protein encoded by the tissue plasminogen activator gene (tPA; Montgomery et al. Cell. Mol. Biol. 1997, 43: 285-292), and the polyadenylation signal (polyA), in particular of the bovine growth hormone (bGH) gene (US-A-5,122,458) or of the rabbit β-globin gene.

Nonlimiting exemplary embodiments of plasmids useful in the invention and exampes are described below, taken with reference to the drawing, in which:
Figure 1.1: plasmid pJP109
Figure 1.2: plasmid pJP111
Figure 1.3: reference plasmid pJP120
Figure 1.4: reference plasmid pJP121
Figure 1.5: reference plasmid pJP058

Sequence listing SEQ ID
   SEQ ID No. 27 oligonucleaotide JP779
   SEQ ID No. 28 oligonucleaotide JP780
   SEQ ID No. 29 oligonucleaotide JP781
   SEQ ID No. 30 oligonucleaotide JP782
   SEQ ID No. 31 oligonucleaotide JP783
   SEQ ID No. 32 oligonucleaotide JP784
   SEQ ID No. 33 oligonucleaotide JP785
   SEQ ID No. 34 oligonucleaotide JP786

### Example 12.1 Construction of the plasmid pJP109

The plasmid pGFM7Z-lmp1010 Stoon-EcoRI No. 14 containing the genome of the PCV-2 virus in the form of an EcoRI fragment (B. Meehan et al. J. Gen. Virol. 1998. 79 2171-2179) was digested with EcoRI in order to isolate, after agarose gel electrophoresis, the EcoRI- EcoRI fragment of 1768 base pairs (bp). This fragment was self-ligated.

The ORF2 gene of the PCV-2 virus strain 1010-Stoon (B. Meehan *et al.* J. Gen. Virol. 1998.79.2171-2179; GenBank sequence accession No. AF055392) was amplified, using the template consisting of the self-ligated EcoRI-EcoRI fragment, by the polymerase chain reaction (PCR) technique with the following oligonucleotides: JP779 (SEQ ID NO 27) (35 mer):
5'CATCATCATGTCGACATGACGTATCCAAGGAGGCG3'
   and JP780 (SEQ ID NO 28) (36 mer):
5TACTACTACAGATCTTTAGGGTTTAAGTGGGGGGTC3'
   in order to generate a 730 bp PCR fragment This fragment was digested with SalI and BglII in order to isolate, after agarose gel electrophoresis, the 715 bp SalI-BglII restriction fragment This fragment was then ligated with the plasmid pVR1012 (Hartikka J. et al. Human Gene Therapy. 1996.7.1205-1217), digested beforehand with SalI and BglII, to give the plasmid pJP109 (5567 pb) (Figure 1.1).

### Example 12.2: Construction of the plasmid PJ111

A polymerase chain reaction was carried out with the plasmid pGem7Z-Imp1010-Stoon (see Example 12.1) (B. Meehan et al. J. Gen. Virol. 1998. 79. 2171-2179), and the following oligonucleotides:
JP781 (SEQ ID NO 29) (35 mer):
5'CATCATCATGTCGACATGCCCAGCAAGAAGAATGG3'
   and JP782 (SEQ ID NO 30) (36 mer):
5'TACTACTACAGATCTTCAGTAATTTATTTCATATGG3'
   in order to generate a 970 bp PCR fragment containing the ORF1 gene of the PCV-2 virus. This fragment was digested with SalI and BglII in order to isolate, after agarose gel electrophoresis, the 955 bp SalI-BglII restriction fragment. This fragment was then ligated with the plasmid pVR1012 (Example 12.1) to give the plasmid pJPIII (5810 bp) (Figure 1.2).

### Reference Example 12.3: Construction of the plasmid pJP120 (PCV-1 ORF2)

A polymerase chain reaction was carried out with the plasmid pPCV1 (B. Meehan et al. J. Gen. Virol. 1997. 78.221-227), and the following oligonucleotides;
JP783 (SEQ ID NO 31) (35 mer):
5'CATCATCATGTCGACATGACGTGGCCAAGGAGGCG3'
   and JP784 (SEQ ID NO 32) (40 mer):
5'TACTACTACAGATCTTTATTTATTTAGAGGGTCTTTTAGG3'
   in order to generate a 730 bp PCR fragment containing the ORF2 gene of the PCV-1 virus (PK-15 strain, GenBank sequence accession No. U49186). This fragment was digested with SalI and BglII in order to isolate, after agarose gel electrophoresis, the 715 bp SalI-BglII restriction fragment. This fragment was then ligated with the plasmid pVR1012 (Example 12.1) to give the plasmid pJP120 (5565 bp) (Figure 1.3).

### Reference Example 12.4: Construction of the plasmid pJP121 (PCV-1 ORF1)

The plasmid pPCV1 containing the PCV1 virus genome in the form of a PstI fragment (B. Meehan et al. J. Gen. Virol. 19,78,221-227) was digested with PstI in order to isolate, after agarose gel electrophoresis, the 1759 base pair (bp) PstI-PstI fragment. This fragment was self-ligated.

The ORF1 gene of the PCV-1 virus strain PK-15 (B. Meehan et al. J. Gen. Virol. 1997, 78, 221-227; GenBank sequence, accession No. U49186) was amplified, using the template consisting of the self-ligated PstI-PstI fragment, by the polymerase chain reaction (PCR) technique with the following oligonucleotides:
JP785 (SEQ ID NO 33) (35 mer):
5'CATCATCATGTCGACATGCCAAGCAAGAAAAGCGG3'
   and JP786 (SEQ ID NO 34) (36 mer):
5'TACTACTACAGATCTTCAGTAATTTATTTTATATGG3'
   in order to generate a 965 bp PCR fragment containing the ORF1 gene of the PCV-1 virus (strain PK-15). This fragment was digested with SalI and BglII in order to isolate, after agarose gel electrophoresis, the 946 bp SalI-BglII restriction fragment. This fragment was then ligated with the plasmid pVR1012 (Example 12.1) to give the plasmid pJP121 (5804 bp) (Figure 1.4).

Reference Example 12.5: Construction of the plasmid pJP058 (expressing porcine GM-CSF)

Pig blood was collected over a tube containing EDTA by taking blood from the jugular vein. The mononucleated cells were harvested by centrifugation on a Ficoll gradient and then cultured *in vitro* in RPMI 1640 medium (Gibco-BRL) and stimulated by addition of concanavaline A (Sigma) at a final concentration of about 5 µg/ml in the culture medium. After 72 hours of stimulation, the lymphoblasts were harvested and the total RNA of these cells was extracted with the extraction kit "Micro-Scale Total RNA Separator Kit" (Clontech) following the manufacturer's recommendations. A reverse transcription reaction, carried out with the aid of the kit "1st-Strand cDNA Synthesis Kit" (Perkin Elmer), followed by a polymerase chain reaction, was carried out on the total RNA extracted from these porcine lymphoblasts with the following oligonucleotides:
RG972 (33 mer):
5'TATGCGGCCGCCAGCATGTGGCTGCAGAACCTG3'
   and RG973 (34 mer):
5'TATGCGGCCGCTACGTATCACTTCTGGGCTGGTT3'
   in order to generate a PCR fragment of about 450 base pairs (bp). This fragment was digested with NotI in order to isolate, after agarose gel electrophoresis, the 450 bp NotI-NotI fragment. This fragment was then ligated with the plasmid pVR1012 (Example 12.1), preferably digested with NotI and dephosphorylated, to give the plasmid pJP058 (5405 bp) (Figure 1.5). The sequence of the pGM-CSF gene cloned into the plasmid pJP058 was checked and found to be identical to that available in the GenBank database (accession No. D21074).

### Example 12.6: Production of the purified plasmids for the vaccination of pigs

*Escherichia coli* K12 bacteria (strains DH10B or SCS1) were transformed with the plasmids pJP109, pJP111, pJP058, pJP120 and pJP121 of Examples 12.1 to 12.5 *supra.* The five transformed clones obtained respectively with these five plasmids were then cultured separately, with shaking at +37°C, in Luria-Broth (LB) medium. The bacterial cultures were harvested at the end of the exponential phase and the plasmids were extracted according to the alkaline lysis technique. The extracted plasmids were then purified on a caesium chloride gradient according to the technique described by Sambrook et al. (Molecular Biology: A Laboratory Manual, 2nd Edition, 1989, Cold Spring Harbor Laboratory, Cold Spring Harbor, NY). After final extraction of ethidium bromide and precipitation in the presence of absolute ethanol, the purified plasmids were resuspended in TE buffer (1 mM Tris/EDTA, pH 8.0) in order to obtain stock solutions containing 2 mg of plasmid per ml. These stock solutions are stored at -20°C before use.

### Example 12.7: Control of the expression of ORFs 1 and 2 of the PCV-2 virus

In order to control the products of expression of the PCV-2 ORF2 and PCV-2 ORF1 genes, cloned respectively into the plasmids pJP109 and pJP111, these plasmids were transfected into CHO-K1 (Chinese Hamster Ovary) cells (ATCC No. CCL-61) with the Lipofectamine Plus®; transfection kit (Gibco-BRL, Catalogue- 10964-013), following the manufacturer's recommendations for use.

48 hours after transfection, the transfected cells are washed and fixed with a 95% glacial acetone solution for 3 minutes at room temperature. Five monoclonal antibodies specific for the PCV-2 ORF1 proteins (F199 1D3GA and F210 7G5GD) and ORF2 proteins (F190 4C7CF, F190 2B1BC and F190 3A8BC) were used as first antibodies. An anti-mouse IgG conjugate, labelled with Cy3, was used to reveal the specific labelling. A PCV-2 specific fluorescence was observed with the 3 PCV-2 ORF2 monoclonals in the cells transfected with the plasmid pJP109, but not in those transfected with the plasmid pJP111. In contrast, a PCV-2 specific fluorescence was observed with the two PCV-2 ORF1 monoclonals in the cells transfected with the plasmid pJP111, but not in those transfected with the plasmid pJP109. No fluorescence was detected with the PCV-2 monoclonals in CHO cells transfected with the plasmid pVR1012 alone or in the nontransfected CHO cells.

The same expression result was obtained with a polyclonal serum specific for the PCV-2 virus. In this case, a fluorescein-labelled anti-pig IgG conjugate was used to detect the specific fluorescence. No fluorescence was detected with this polyclonal serum in CHO cells transfected with the plasmid pVR1012 alone or in the nontransfected CHO cells.

### Reference Example 12.8: Vaccination of pigs with naked DNA

### 12.8.1. One-day-old piglets

Groups of piglets obtained by Caesarean on DO of the protocol, are placed in an isolating unit. These piglets are vaccinated at the age of 2 days by the intramuscular route with various vaccinal solutions of plasmid. The vaccinal solutions are prepared by diluting the stock solutions in sterile physiological saline (0.9% NaCl).

The piglets are vaccinated:
either with the plasmid pJP109 alone
or with the mixture of the plasmids pJP109 and pJP111
or with the mixture of the plasmids pJP109 and reference plasmid pJP058
or with the mixture of the plasmids pJP109, pJP111 and reference plasmid pJP058

The vaccinal solutions comprise 500 µg of each plasmid. Volume: The vaccinal solutions are injected by the intramuscular route in a total volume of 2 ml. In practice, given the age of the piglets on vaccination (1-2 days), 1 injection of 1 ml is given on each side of the neck (= 2 x 1 ml).

Two injections of vaccine are carried out at two weeks' interval, that is to say on days D2 and D14 of the protocol.

A challenge is made on D21 of the protocol by oronasal administration of a viral suspension of a virulent PCV-2 strain. The piglets are then monitored for 3 weeks for the appearance of specific clinical signs of post-weaning multisystemic wasting syndrome in piglets. The signs which are monitored are:
rectal temperature: daily measurement for the first 14 days, then two measurements during the 3rd week following the challenge.
Weight: weighing of the piglets just before the challenge then once per week during the 3 weeks following the challenge.
Collection of blood samples to test for: viremia and antibodies: blood samples taken on D2, D14, D21, D28, D35 and D42.
Autopsy: on D42, the surviving pigs are humanely killed and undergo autopsy to search for anatomicopathological lesions and to make histological preparations from the liver, the lymph nodes, the spleen, the kidneys and the thymus to search for lesions in these tissues.

### 12.8.2. 5-7-week old piglets

5- to 7-week old piglets, no longer having maternal antibodies specific for the PCV-2 virus are vaccinated by the intramuscular route:
either with the plasmid pJP109 alone,
or with the mixture of the plasmids pJP109 and pJP111
or with the mixture of the plasmids pJP109 and reference plasmid pJP058
or with the mixture of the plasmids pJP109, pJP111 and reference plasmid pJP058
   the vaccinal doses are the same as those indicated in Example 12.8.1 (500 µg per plasmid). The vaccinal solutions are injected by the intramuscular route in a volume of 2 ml (a single administration of 2 ml, into the neck muscles).

Two vaccinations are performed at 21 days interval (DO and D21). A challenge is made 14 days after the last vaccination (D35) by intramuscular administration of a viral suspension of a virulent PCV-2 strain.

The pigs are then monitored for 8 weeks for the occurrence of specific clinical signs of the post-weaning multisystemic wasting syndrome in piglets. The clinical monitoring of the piglets after the challenge is identical to that described in Example 12.8.1 except that the total duration of observation is this time 8 weeks.

### Reference Example 12.9: Vaccination of pigs with DNA formulated with DMRIE-DOPE

It is possible to use, in place of the naked plasmid DNA solutions described in Example 12.8 solutions of
plasmid DNA. formulated with DMRIE-DOPE. A DNA solution (containing one or more plasmids according to Example 12.6) at 1 mg/ml is prepared in 0.9% NaCl. A DMRIE-DOPE solution at 0.75 mM is prepared by taking up a lyophilisate of DMRIE-DOPE in a suitable volume of sterile distilled water.

The formation of the plasmid DNA-cationic lipid complexes is achieved by diluting, in equal parts, the DMRIE-DOPE solution at 0.75 mM with the DNA solution at 1 mg/ml in 0.9% NaCl. The DNA solution is introduced gradually, with the aid of a syringe mounted with a 26G needle, along the wall of the vial containing the cationic lipid solution so as to avoid the formation of foam. Gentle shaking is carried out as soon as the two solutions have been mixed. A composition comprising 0.375 mM DMRIE-DOPE and 500 µg/ml of DNA is finally obtained.

It is desirable for all the solutions used to be at room temperature for all the operations described above. The DNA/DMRIE-DOPE complex formation is performed at room temperature for 30 minutes before immunizing the pigs.

The pigs are then vaccinated according to the conditions described in Examples 12.8.1. and 12.8.2.

### Porcine Circovirus Recombinant Poxvirus Vaccine Reference Example 13

The following passages are extracts from US Application No. 60/138478.

This example refers to ORF1 (Meehan *et al.*, 1998; corresponding to COL4 in the French patent application 98 03707), comprising nt 398-1342 (GenBank accession number AF055392), has the potential to encode a protein with a predicted molecular weight of 37.7 kD. ORF2 (Meehan *et al.,* 1998; corresponding to COL13 in the French patent application 98 03707), comprising nt 1381-1768 joined to 1-314 (GenBank accession number AF055392), may encode a protein with a predicted molecular weight of 27.8 kD.

A better understanding of this example will be had by referring to the accompanying drawings, in which:
- FIG. 2.1 (SEQ ID NO: 16) shows the refernce nucleotide sequence of a 3.7 kilobase pair fragment of ALVAC DNA containing the C6 open reading frame.

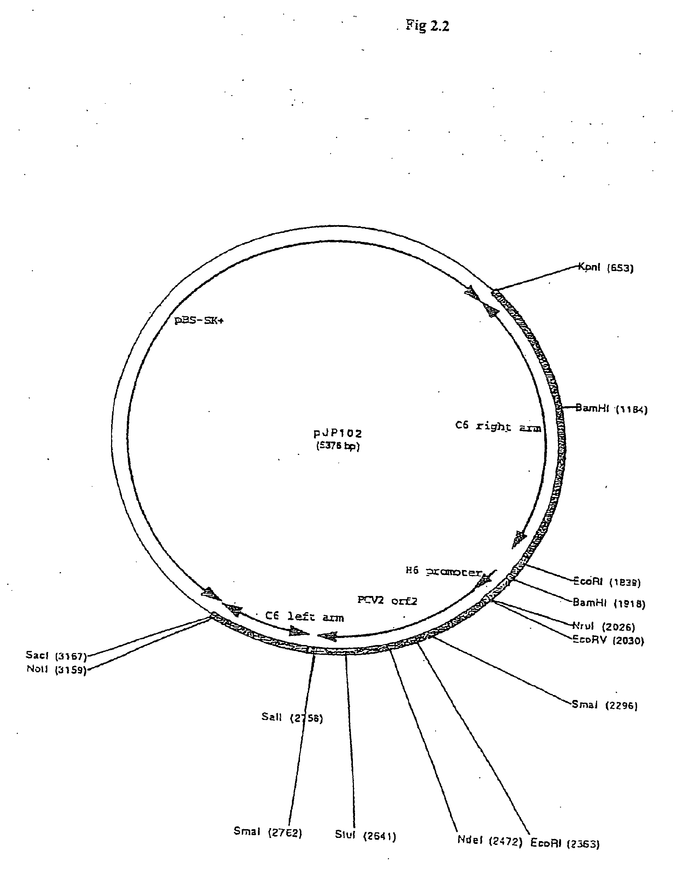

- FIG. 2.2 shows the map of pJP102 donor plasmid.

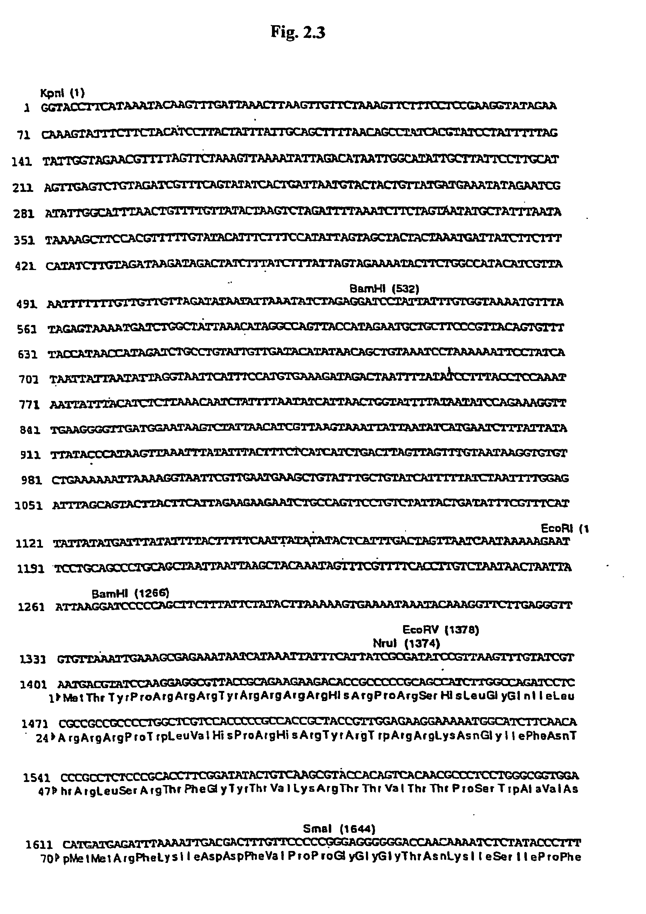

- FIG. 2.3 (SEQ ID NO: 8) shows the nucleotide sequence of a 2.5 kilobase pair fragment from pJP102 donor plasmid from the *KpnI* (position 653) to the *SacI* (position 3166) restriction sites.

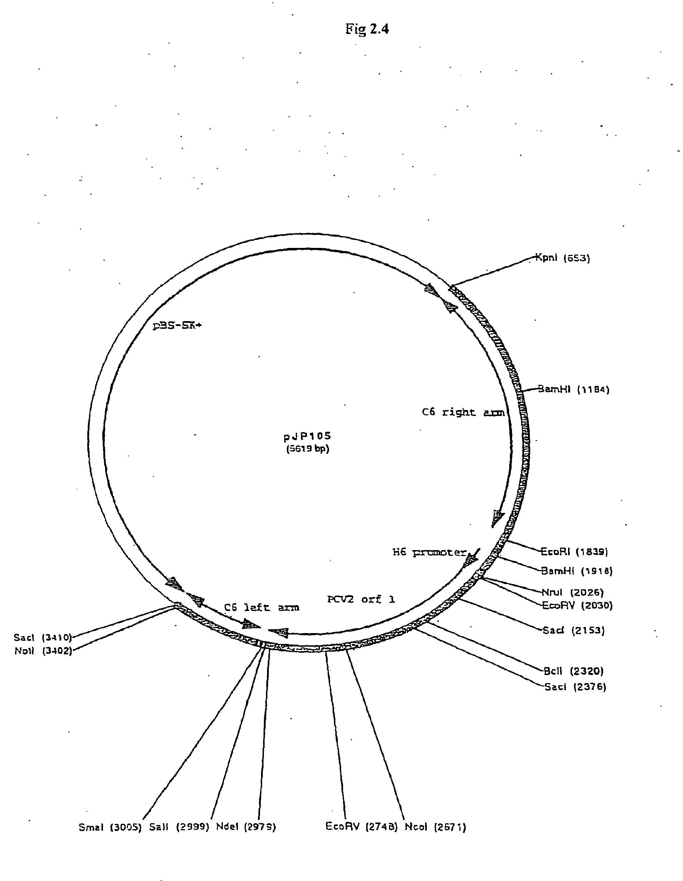

- FIG. 2.4 shows the map of pJP 105 donor plasmid.

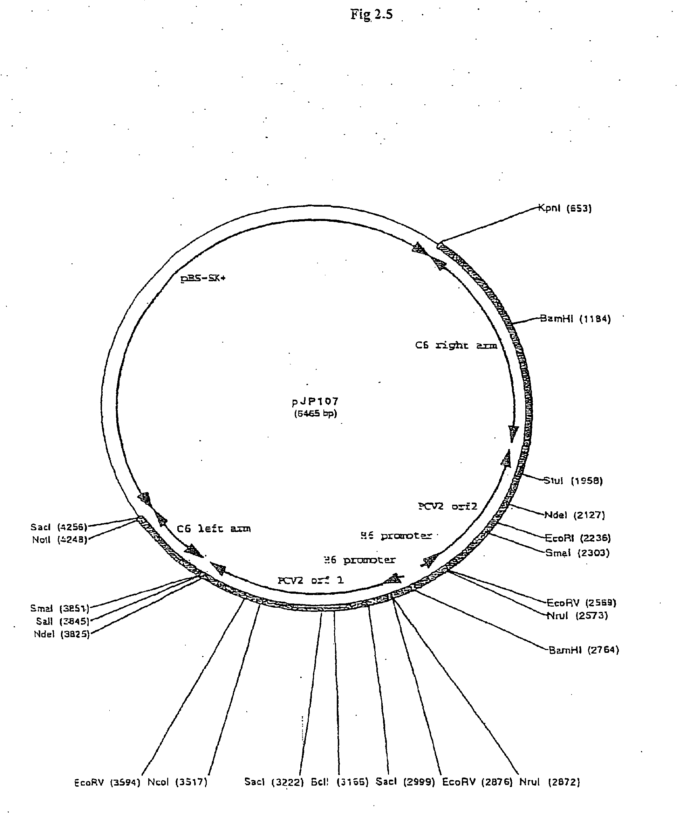

- FIG. 2.5 shows the map of reference pJP 107 donor plasmid.

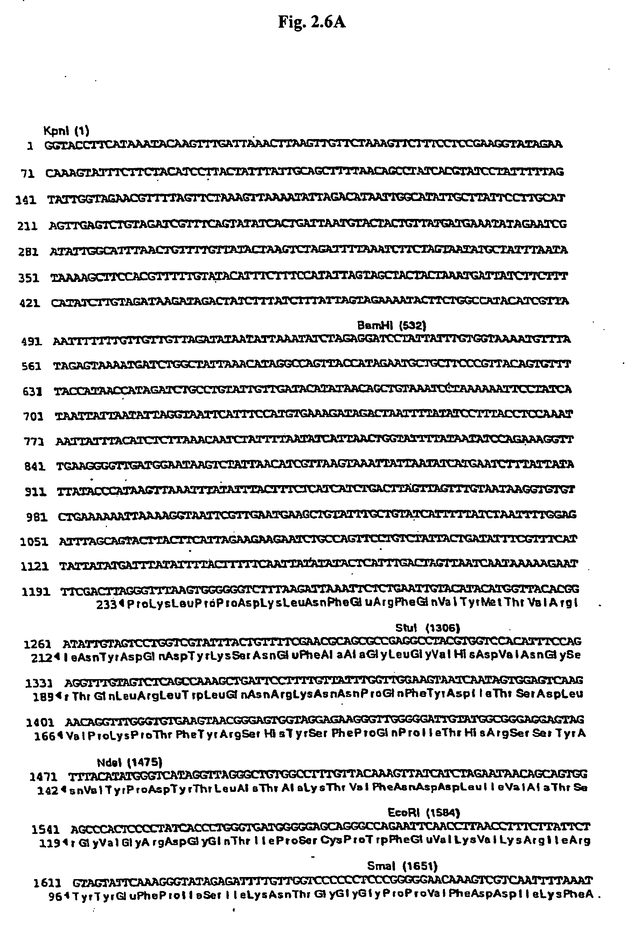

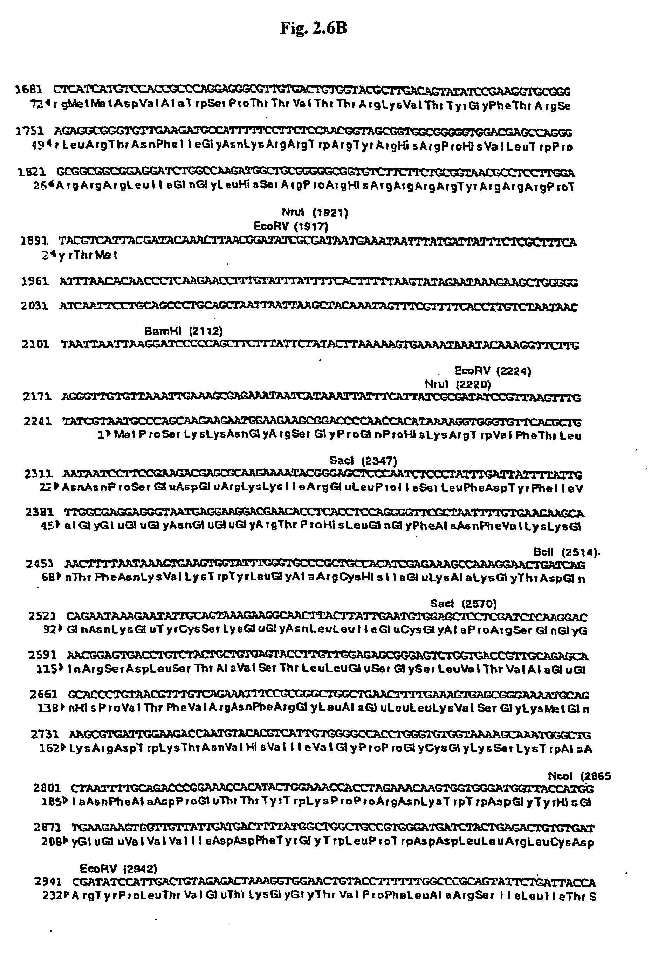

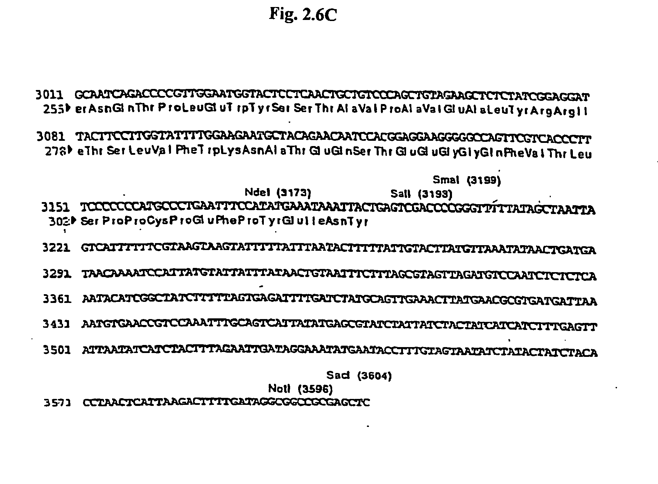

- FIG. 2.6 (SEQ ID NO: 11) shows the reference nucleotide sequence of the 3.6 kilobase pair fragment from pJP107 donor plasmid from the *KpnI* (position 653) to the *SacI* (position 4255) restriction sites.

We describe recombinant poxviruses containing therein a DNA sequence from PCV2 in a nonessential region of the poxvirus genome. The recombinant poxviruses express gene products of the foreign PCV2 gene. In particular, ORF2 and ORF1 genes encoding PCV2 proteins were isolated, characterized and inserted into ALVAC (canarypox vector) recombinants. The molecular biology techniques used are the ones described by Sambrook et al. (1989). Cell Lines and Virus Strains. The strain of PCV2 designated Imp. 1010-Stoon has been previously described (Meehan et al., 1998). It was isolated from mesenteric lymph node tissues from a diseased pig originating from Canada. Cloning of the PCV2 genome was described by Meehan et al. (1998). Plasmid pGem7Z-1mp1010- Stoon-EcoRI No. 14 contains the PCV2 genome as an *Eco*RI fragment inserted into the *Eco*RI site of plasmid pGem-7Z (Promega, Madison, WI). The complete nucleotide sequence of the Imp. 1010-Stoon PCV2 strain has been determined by Meehan et al. (1998) and is available under the GenBank accession number AF055392.

The parental canarypox virus (Rentschler strain) is a vaccinal strain for canaries. The vaccine strain was obtained from a wild type isolate and attenuated through more than 200 serial passages on chick embryo fibroblasts. A master viral seed was subjected to four successive plaque purifications under agar and one plaque clone was amplified through five additional passages after which the stock virus was used as the parental virus in *in vitro* recombination tests. The plaque purified canarypox isolate is designated ALVAC. ALVAC was deposited November 14, 1996 under the terms of the Budapest Treaty at the American Type Culture Collection, ATCC accession number VR-2547.

The generation of poxvirus recombinants involves different steps: (1) construction of an insertion plasmid containing sequences ("arms") flanking the insertion locus within the poxvirus genome, and multiple cloning site (MCS) localized between the two flanking arms (e.g., see Reference Example 13.1); (2) construction of donor plasmids consisting of an insertion plasmid into the MCS of which a foreign gene expression cassette has been inserted (e.g. see Examples 13.2 to 13.5); (3) *in vitro* recombination in cell culture between the arms of the donor plasmid and the genome of the parental poxvirus allowing the insertion of the foreign gene expression cassette into the appropriate locus of the poxvirus genome, and plaque purification of the recombinant virus (e.g. see Example 13.6).

### Reference Example 13.2 - CONSTRUCTION OF CANARYPOX INSERTION PLASMID AT C6 LOCUS

Figure 2.1 (SEQ ID NO: 16) is the sequence of a 3.7 kb segment of canarypox DNA. Analysis of the sequence revealed an ORF designated C6L initiated at position 377 and terminated at position 2254. The following describes a C6 insertion plasmid constructed by deleting the C6 ORF and replacing it with a multiple cloning site (MCS) flanked by transcriptional and translational termination signals. A 380 bp PCR fragment was amplified from genomic canarypox DNA using oligonucleotide primers C6A1 (SEQ ID NO: 21) and C6B 1 (SEQ ID NO: 22). A 1155 bp PCR fragment was amplified from genomic canarypox DNA using oligonucleotide primers C6CI (SEQ ID NO: 23) and C6D1 (SEQ ID NO: 24). The 380 bp and 1155 bp fragments were fused together by adding them together as template and amplifying a 1613 bp PCR fragment using oligonucleotide primers C6A1 (SEQ ID NO: 21) and C6D1 (SEQ ID NO: 24). This fragment was digested with S*ac*I and K*pn*I, and ligated into pBluescript SK+ (Stratazene, La Jolla, CA, USA) digested with *Sac*I/*Kpn*I. The resulting plasmid, pC6L was confirmed by DNA sequence analysis. It consists of 370 bp of canarypox DNA upstream of C6 ("C6 left arm"), vaccinia early termination signal, translation stop codons in six reading frames, an MCS containing S*ma*I, P*st*I, X*ho*I and EcoRI sites, vaccinia early termination signal, translation stop codons in six reading frames and 1156 bp of downstream canary pox sequence ("C6 right arm").

Plasmid pJP099 was derived from pC6L by ligating a cassette containing the vaccinia H6 promoter (described in Taylor et al. (1988c), Guo et al. (1989), and Perkus et al. (1989)) coupled to a foreign gene into the S*ma*I/E*co*RI sites of pC6L.

This plasmid pJP099 contains a unique E*co*RV site and a unique N*ru*I site located at the 3' end of the H6 promoter, and a unique *SalI* site located between the STOP codon of the foreign gene and the C6 left arm. The -4.5 kb E*co*RVIS*al*I or N*ruI*S*al*I fragment from pJP099 contains therefore the plasmid sequence (pBluescript SK+ ; Stratagene, La Jolla, CA, USA), the 2 C6 arms and the 5' end of the H6 promoter until the EcoRV or *Nrul* site.

Sequences of the primers:
Primer C6A1 (SEQ ID NO:21)
   ATCATCGAGCTCGCGGCCGCCTATCAAAAGTCTTAATGAGTT
Primer C6B1 (SEQ ID NO: 22) 
GAATTCCTCGAGCTGCAGCCCGGGTTTTTATAGCTAATTAGTCATTTTTTC
GTAAGTAAGTATTTTTATTTAA
Primer G6C1 (SEQ ID NO: 23) 
CCCGGGCTGCAGCTCGAGGAATTCTTTTTATTGATTAACTAGTCAAATGAG
TATATATAATTGAAAAAGTAA
Primer C6D1 (SEQ ID NO: 24)
   GATGATGGTACCTTCATAAATACAAGTTTGATTAAACTTAAGTTG

### Example 13.2 - CONSTRUCTION OF ALVAC DONOR PLASMID FOR PCV2 ORF2

Plasmid pGem 7Z-Imp1010-Stoon-EcoRI No. 14, containing the PCV2 genome as an *Eco*RI fragment in plasmid pGem-7Z, was digested with *Eco*RI, and a 1768bp fragment was isolated and ligated.

In order to insert PCV2 ORF 2 into an appropriate ALVAC insertion vector: Primers JP760 (SEQ ID NO: 25) and JP773 (SEQ ID NO: 26) were used to amplify PCV2 ORF 2 from the 1768bp ligated *Eco*RI fragment (see above) resulting in PCR J1304. Primer JP760 (SEQ ID NO: 25) contains the 3' end of the H6 promoter from *Eco*RV and the 5' end of PCV2 ORF 2. Primer JP773 (SEQ ID NO: 26) contains the 3' end of PCV2 ORF 2 followed by a *Sal*I site. The product of PCR J1304 was then digested with *Eco*RV/*Sal*I and cloned as a-750 bp fragment into a-4.5 kb *Eco*RV/SalI fragment from pJP099 (see above in Example 13.1). The resulting plasmid was confirmed by sequence analysis and designated pJP102 (see the map of pJP102 in Figure 2.2 and the sequence (SEQ ID NO: 8) in Figure 2.3). The sequence of ORF 2 matches sequence available in GenBank, Accession Number AF055392. The donor plasmid pJP102 (linearized with *Not*I) was used in an *in vitro* recombination (IVR) test to generate ALVAC recombinant vCP1614 (see Example 13.6).

Sequence of the primers:
JP760 (SEQ ID NO: 25) 
CAT-CAT-CAT-GAT-ATC-CGT-TAA-GTT-TGT-ATC-GTA-ATG-ACG-TAT- CCA-
AGG-AGG-CG
JP773 (SEQ ID NO: 26)
   TAC-TAC-TAC-GTC-GAC-TTA-GGG-TTT-AAG-TGG-GGG-GTC

### Reference Example 13.3 - CONSTRUCTION OF AN ALVAC DONOR PLASMID FOR PCV2 ORF2 AND ORF1

PCV2 ORF 1 was amplified by PCR using primers JP774 (SEQ ID NO: 9) and JP775 (SEQ ID NO: 10) on plasmid pGem 7Z-Imp1010-Stoon-EcoRI No. 14 resulting in PCR J1311. Primer JP774 (SEQ ID NO: 9) contains the 3' end of the H6 promoter from *NruI* and the 5' end of PCV2 ORF1. Primer JP775 (SEQ ID NO: 10) contains the 3' end of PCV2 ORF1 followed by a *Sal*I site. The product of PCR J1311 (-1 Kb) was cloned into pCR2.1 (Invitrogen, Carlsbad, CA). The resulting plasmid was confirmed by sequence analysis and designated pJP104. The sequence of ORF1 matches sequence available in GenBank, Accession Number AF055392. A-970 bp *Nru*I/*Sal*I fragment was isolated from pJP104 and cloned into a-4.5 kb *Nru*I/*Sal*I fragment from pJP099 (see Example 13.1), resulting in a plasmid which was confirmed by restriction analysis and designated pJP105 (see Figure 2.4). The donor plasmid pJP105 could be used in an *in vitro* recombination test (described in Example 13.6) to generate ALVAC recombinant expressing the PCV2 ORF1.

A-838bp *Bam*HI/*Sal*I from pJP102 (see Example 13.2) was blunted using the Klenow fragment of DNA polymerase, and was cloned into the Klenow-blunted *Eco*RI site of pJP105. Clones were checked for orientation of insert by restriction analysis and a head-to-head orientation was chosen. This plasmid was confirmed by sequence analysis and designated pJP107 (see the map of pJP107 in Figure 2.5 and the sequence (SEQ ID NO: 11) in Figure 2.6). The donor plasmid pJP107 (linearized with *Not*I) was used in an *in vitro* recombination 5 (IVR) test to generate the ALVAC recombinant vCP1615 (see Example 13.6).

Sequence of the primers.
JP774 (SEQ ID NO: 9) 
CAT-CAT-CAT-TCG-CGA-TAT-CCG-TTA-AGT-TTG-TAT-CGT-AAT-GCC- CAG-
CAA-GAA-GAA-TGG
JP775 (SEQ ID NO: 10)
   TAC-TAC-TAC-GTC-GAC-TCA-GTA-ATT-TAT-TTC-ATA-TGG

### Reference Example 13.4 - CONSTRUCTION OF ALVAC DONOR PLASMID FOR PCV1 ORF2

Plasmid pPCV1 (B. Meehan *et al.* J. Gen. Virol. 1997.78.221-227), containing the PCV1 genome as a *Pst*I fragment in plasmid pGem-7Z, was used as a template to amplify the PCV1 ORF2.

In order to insert PCV2 ORF 2 into an appropriate ALVAC insertion vector : Primers JP787 (SEQ ID NO: 12) and JP788 (SEQ ID NO: 13) were used to amplify PCV1 ORF 2 from plasmid pPCV1 (see above) resulting in PCR J 131.5. Primer JP787 (SEQ ID NO: 12) contains the 3' end of the H6 promoter from *Eco*RV and ORF 2 followed by a *Sal*I site. The product of PCR J1315 was then digested with *Eco*RV/*Sal*I and cloned as a-750 bp fragment into a-4.5 kb *Eco*RV/*Sal*I fragment from pJP099 (see above in Example 13.1). The resulting plasmid was confirmed by sequence analysis and designated pJP 113. The sequence of ORF 2 matches sequence available in GenBank, Accession Number U49186. The donor plasmid pJP113 (linearized with *Not*I) was used in an *in vitro* recombination (IVR) test to generate ALVAC recombinant vCP1621 (see Example 13.7).

Sequence of the primers.
JP787 (SEQ ID NO: 12) 
CAT-CAT-CAT-GAT-ATC-CGT-TAA-GTT-TGT-ATC-GTA-ATG-ACG-TGG- CCA-
AGG-AGG-CG
JP788 (SEQ ID NO: 13)
   TAC-TAC-TAC-GTC-GAC-TTA-TTT-ATT-TAG-AGG-GTC-TTT-TAG-G

### Reference Example 13.5 - CONSTRUCTION OF AN ALVAC DONOR PLASMID FOR PCV1 ORF2 AND ORFI

Plasmid pPCV1 (see Example 13.4 above), containing the PCV1 genome as a *Pst*I fragment in plasmid pGem-7Z, was digested with *Pst*I, and a 1759 bp fragment was isolated and ligated.

Primers JP789 (SEQ ID NO: 14) and JP790 (SEQ ID NO: 15) were used to amplify PCV1 ORF1 from the 1759 bp ligated *Pst*I fragment (see above), resulting in PCR J1316. Primer JP789 (SEQ ID NO: 14) contains the 3' end of the H6 promoter from *Nru*I and the 5' end of PCV 1 ORF1. Primer JP790 (SEQ ID NO: 15) contains the 3' end of PCV1 ORF1 followed by a *Sal*I site. The product of PCR J1316 (-1 Kb) was cloned into pCR2.1 (Invitrogen, Carlsbad, CA). The resulting plasmid was confirmed by sequence analysis and designated pJP114. The sequence of ORF1 matches sequence available in GenBank, Accession Number U49186. A -970 bp *Nru*I/*Sal*I fragment was isolated from pJP114 and cloned into a -4.5 kb *Nru*I/*Sal*I fragment from pJP099 (see Example 13.1), resulting in a plasmid which was confirmed by restriction analysis and designated pJP115. The donor plasmid pJP115 could be used in an in vitro recombination test (described in Example 13.7) to generate ALVAC recombinant expressing the PCV 1 ORF1.

A -838bp *Bam*HI/*Sal*I from pJP 113 (see Example 2.4) was blunted using the Klenow fragment of DNA polymerase, and was cloned into the Klenow-blunted *Eco*RI site of pJP115. Clones were checked for orientation of insert by restriction analysis and a head-to-head orientation was chosen. This plasmid was confirmed by sequence analysis and designated pJP117. The donor plasmid pJP117 (linearized with *Not*I) was used in an *in vitro* recombination (IVR) test to generate the ALVAC recombinant vCP1622 (see Example 13.7).

Sequence of the primers.
JP789 (SEQ ID NO: 14) 
CAT-CAT-CAT-TCG-CGA-TAT-CCG-TTA-AGT-TTG-TAT-CGT-AAT-GCC- AAG-
CAA-GAA-AAG-CGG
JP790 (SEQ ID NO: 15)
   TAC-TAC-TAC-GTC-GAC-TCA-GTA-ATT TAT-TTT-ATA-TGG

### Reference Example 2.6 - GENERATION OF ALVAC-PCV2 RECOMBINANTS

Plasmids pJP102 (see Example 13.2 and Figure 2.2) and reference plasmid pJP107 (see Example 13.3 and Figure 2.5) were linearized with *Not*I and transfected into ALVAC infected primary CEF cells by using the calcium phosphate precipitation method previously described (Panicali and Paoletti, 1982; Piccini et al., 1987). Positive plaques were selected on the basis of hybridization to specific PCV2 radiolabeled probes and subjected to four sequential rounds of plaque purification until a pure population was achieved. One representative plaque from each IVR was then amplified and the resulting ALVAC recombinants were designated vCP1614 and vCP1615. The vCP1614 virus is the result of recombination events between ALVAC and the donor plasmid pJP 102, and it contains the PCV2 ORF2 inserted into the ALVAC C6 locus. The vCP1615 virus is the result of recombination events between ALVAC and the donor plasmid pJP107, and it contains the PCV2 ORF2 and ORF1 inserted into the ALVAC C6 locus in a head-to-head orientation.

In a similar fashion, a recombinant ALVAC expressing only PCV2 ORF1 can be generated using the donor plasmid pJP105 described in Example 13.3.

Immunofluorescence. In order to determine if the PCV2 proteins were expressed in ALVAC recombinant infected Vero cells, immunofluorescence (IF) analysis was performed. Infected Vero cells were washed with PBS 24 hrs after infection (m.o.i. of approx. 10) and fixed with 95% cold aceton for 3 minutes at room temperature. Five monoclonal antibody (MAb) preparations (hybridoma supermatant) specific for PCV2 ORF1 (PCV2 199 1D3GA & PCV2 210 7G5GD) or ORF2 (PCV2 190 4C7CF, PCV2 190 2B1BC & PCV2 190 3A8BC) were used as the first antibody. These specific monoclonal antibodies were obtained from Merial-Lyon. Monoclonal antibodies can also be obtained following the teachings of documents cited herein, e.g. U.S. Ser. Nos. 09/082,358 and 09/161,092, incorporated herein by reference. The IF reaction was performed as described by Taylor et al. (1990).

PCV2 specific immunofluorescence with the three ORF2-specific antibodies could be detected in cells infected with vCP1614 and cells infected with vCP1615. PCV2 specific immunofluorescence with the two ORF1-specific antibodies could be detected in cells infected with vCP1615 only. These results indicated that, as expected, vCP1614 expresses only ORF2, whereas vCP1615 expresses both ORF1 and ORF2. No fluorescence was detected in parental ALVAC infected Vero cells, nor in uninfected Vero cells.

**Reference Example 13.7 - GENERATION OF ALVAC-PCV1 RECOMBINANTS** Plasmids pJP113 (see Example 13.4) and pJP117 (see Example 13.5) were linearized with *Not*I and transfected into ALVAC infected primary CEF cells by using the calcium phosphate precipitation method previously described (Panicali and Paoletti, 1982; Piccini et al., 1987). Positive plaques were selected on the basis of hybridization to specific PCV1 radiolabeled probes and subjected to four sequential rounds of plaque purification until a pure population was achieved. One representative plaque from each IVR was then amplified and the resulting ALVAC recombinants were designated vCP1621 and vCP1622. The vCP1621 virus is the result of recombination events between ALVAC and the donor plasmid pJP113, and it contains the PCV1 ORF2 inserted into the ALVAC C6 locus. The vCP1622 virus is the result of recombination events between ALVAC and the donor plasmid pJP 117, and it contains the PCV1 ORF2 and ORF1 inserted into the ALVAC C6 locus in a head-to-head orientation.

In a similar fashion, a recombinant ALVAC expressing only PCV1 ORF1 can be generated using the donor plasmid pJP 15 described in Example 13.5.

Immunofluorescence. In order to determine if the PCV1 proteins were expressed in ALVAC recombinant infected Vero cells, immunofluorescence (IF) analysis was performed. Infected Vero cells were washed with PBS 24 hrs after infection (m.o.i. of approx. 10) and fixed with 95% cold aceton for 3 minutes at room temperature. A specific anti-PCVI pig polyclonal serum (Allan G. et al. Vet Microbiol. 1999.66: 115-123) was used as the first antibody. The IF reaction was performed as described by Taylor et al. (1990).

PCV1 specific immunofluorescence could be detected in cells infected with vCP1621 and cells infected with vCP1622. These results indicated that, as expected, vCP1621 and vCP1622 express PCV1-specific products. No fluorescence was detected with a PCV2-specific pig polyclonal serum in cells infected with vCP1621 and in cells infected with vCP1622. No fluorescence was detected in parental ALVAC infected Vero cells, nor in uninfected Vero cells.

### Reference Example 13.8: FORMULATION OF RECOMBINANT CANARYPOX VIRUSES WITH CARBOPOL^{™} 974P

For the preparation of vaccines, recombinant canarypox viruses vCP1614 and vCP1615 (Example 13.6) can be mixed with solutions of carbomer. In the same fashion, recombinant canarypox viruses vCP1621 and vCP1622 (Example 13.7) can be mixed with solutions of carbomer. The carbomer component used for vaccination of pigs according to the present invention is the Carbopol^{™} 974P manufactured by the company BF Goodrich (molecular weight of #3,000,000). A 1.5 % Carbopol^{™} 974P stock solution is first prepared in distilled water containing 1 g/l of sodium chloride. This stock solution is then used for manufacturing a 4 mg/ml Carbopol^{™} 974P solution in physiological water. The stock solution is mixed with the required volume of physiological water, either in one step or in several successive steps, adjusting the pH value at each step with a IN (or more concentrated) sodium hydroxide solution to get a final pH value of 7.3-7.4. This final Carbopol^{™} 974P solution is a ready-to-use solution of reconstituting a lyophilized recombinant virus or for diluting a concentrated recombinant virus stock. For example, to get a final viral suspension containing 10°8 pfu per dose of 2 ml, one can dilute 0,1 ml of a 10°9 pfu/ml stock solution into 1,9 ml of the above Carbopol^{™} 974P 4 mg/ml ready-to-use solution. In the same fashion, Carbopol^{™} 974P 2 mg/ml ready-to-use solutions can also be prepared.

### Reference Example 13.9 - IMMUNIZATION OF PIGS AND SUBSEQUENT CHALLENGE 13.9.1. IMMUNIZATION OF 1 DAY-OLD PIGLETS

Groups of piglets, caesarian-derived at Day 0, are placed into isolators. The piglets are vaccinated by intramuscular route at Day 2 with various vaccine solutions. Vaccine viral suspensions are prepared by dilution of recombinant viruses stocks in sterile physiological water (NaCl 0.9 %). Suitable ranges for viral suspensions can be determined empiracally, but will generally range from 10⁶ to 10¹⁰ and preferably about 10¹⁰, pfu/dose. Vaccine solutions can also be prepared by mixing the recombinant virus suspension with a solution of Carbopol^{™} 974P, as described in Example 13.8.

Piglets are vaccinated either with:
Recombinant virus vCP1614 (Example 13.2)
Recombinant virus vCP1615 (Example 13.3)
Recombinant virus vCP1614 mixed with Carbopol (4 mg/ml solution)
Recombinant virus vCP1615 mixed with Carbopol (4 mg/ml solution).

The viral suspensions contain 10°8 plaque forming units (pfu) per dose. Each viral suspension is injected by intramuscular route under a volume of 1 ml. The intramuscular injection is administered into the muscles of the neck.

Two injections of viral suspensions are administered at Day 2 and Day 14 of the experiment. A challenge is done on Day 21 by an oronasal administration of a viral suspension prepared from a culture of PCV-2 virulent strain. After challenge, piglets are monitored during 3 weeks for clinical signs specific of the post-weaning multisystemic syndrome. The following signs are scored :
Rectal temperature : daily monitoring for 2 weeks post-challenge, then 2 measures of rectal temperature during the third week.
Weight: piglets are weighed right before the challenge, and then weekly during the first 3 weeks post-challenge.
Blood samples are taken at Day 2, Day 14, Day 21, Day 28, Day 35 and Day 42 of the experiment in order to monitor viremia levels and anti-PCV-2 specific antibody titers.
Necropsies : at Day 42, all surviving piglets are humanely euthanized and necropsied to look for specific PWMS macroscopic lesions. Tissue samples are prepared from liver, lymph nodes, spleen, kidneys and thymus in order to look for specific histological lesions.

### 13.9.2. IMMUNIZATION OF 5-7 WEEK-OLD PIGLETS

5-7 week-old piglets, free of anti-PCV-2 specific maternal antibodies, are vaccinated by intramuscular route with various vaccine solutions. Vaccine viral suspensions are prepared by dilution of recombinant viruses stocks in sterile physiological water (NaCl 0.9 %). Vaccine solutions can also be prepared by mixing the recombinant virus suspension with a solution of Carbopol^{™} 974P, as described in Example 13.8.

Piglets are vaccinated either with:
Recombinant virus vCP1614 (Example 13.2)
Recombinant virus vCP1615 (Example 13.3)
Recombinant virus vCP1614 mixed with Carbopol (4 mg/ml solution)
Recombinant virus vCP1615 mixed with Carbopol (4 mg/ml solution)

The viral suspensions contain 10°8 plaque forming units (pfu) per dose. Each viral suspension is injected by intramuscular route under a volume of 2 ml. The intramuscular injection is administered into the muscles of the neck.

Two injections of the viral suspensions are administered at Day 0 and Day 21 of the experiment. A challenge is done at Day 35 by an oronasal administration of a viral suspension prepared from a culture of PCV-2 virulent strain. After challenge, piglets are monitored during 8 weeks for clinical signs specific of the post-weaning
multisystemic syndrome. The clinical monitoring is identical to the one described in Example 13.9.1. except that total duration of monitoring is 8 weeks instead of 3 weeks.

Necropsies are done throughout the experiment for piglets dying from the challenge and at the end of the experiment (Day 97) for all surviving piglets. Tissue samples are the same as described in Example 13.9.1.

### REFERENCES

1. Clark, E. G. Proc. Amer. Assoc. Swine Pract, pp. 499-501 (1997).
2. Edbauer, C., R. Weinberg, J. Taylor, A. Rey-Senelonge, J. F. Bouquet, P. Desmettre and E. Paoletti, Virology 179, 901-904 (1990).
3. Ellis, J., L. Hassard, E. Clark, J. Harding, G. Allan, P. Willson, J. Strakappe, K. Martin, F. McNeilly, B. Meehan, D. Todd, D. Haines, Can. Vet. J. 39, 44-51 (1998).
4. Goebel, S. J., G. P. Johnson, M. E. Perkus, S. W. Davis, J. P. Winslow, E. Paoletti, Virology 179, 247-266, 517-563 (1990).
5. Guo, P., S. Goebel, S. Davis, M. E. Perkus, B. Languet, P. Desmettre, G. Allen, and E. Paoletti, J. Virol. 63, 4189-4198 (1989).
6. Hamel, A. L., L. L. Lin and G. P. S. Nayar, J. Virol. 72, 5262-5267 (1998).
7. Harding J. C., Proc. Am. Assoc. Swine Pract 28, 503 (1997).
8. Mankertz, A., J. Mankertz, K. Wolf, H.-J. Buhk, Gen. Virol. 79, 381-384 (1998a).
9. Mankertz, J., H.-J. Buhk, G. Blaess, A. Mankertz, Virus Gene 16, 267-276 (1998b).
10. Matthews, R. E. F., Intervirology 17, 42-44 (1982).
11. Meehan, B. M., J. L. Creelan, M. S. McNulty, D. Todd, J. Gen. Virol. 78, 221-227 (1997).
12. Meehan, B. M., F. McNeilly, D. Todd, S. Kennedy, V. A. Jewhurst, J. A. Ellis, L. E. Hassard, E. G. Clark, D. M. Haines, G. M. Allan, J. Gen. Virol. 79, 2171-2179 (1998).
13. Nayar, G. P. S., A. Hamel and L. Lin. Can. Vet. J. 38, 385-386 (1997).
14. Panicali, D. and E. Paoletti, Proc. Natl. Acad. Sci. USA 79, 4927-4931 (1982).
15. Paoletti, E., B. R. Lipinskaks, C. Samsonoff, S. Mercer, and D. Panicali, Proc. Natl. Acad. Sci. U.S.A. 81, 193-197 (1984).
16. Perkus, M. E., K. Limbach, and E. Paoletti, J. Virol. 63, 3829-3836 (1989).
17. Piccini, A., M. E. Perkus, and E. Paoletti, In Methods in Enzymology, Vol. 153, eds. Wu, R., and Grossman, L., (Academic Press) pp. 545-563 (1987).
18. Sambrook, J., E. F. Fritsch, and T. Maniatis, In Molecular cloning: A laboratory manual, 2nd edition, (Cold Spring Harbor Press, NY) (1989).
19. Tartaglia, J., J. Winslow, S. Goebel, G. P. Johnson, J. Taylor, and E. Paoletti, J. Gen. Virol. 71, 1517-1524 (1990).
20. Tartaglia, J., Perkus ME, Taylor J, Norton EK, Audonnet JC, Cox WI, Davis SW, van der Hoeven J, Meignier B, Riviere M, and E. Paoletti, Virology 188, 217-232 (1992).
21. Taylor, J., R. Weinberg, B. Languet, Ph. Desmettre and E. Paoletti, Vaccine 6, 497- 503 (1988a).
22. Taylor, J. and E. Paoletti, Vaccine 6, 466-468 (1988b).
23. Taylor, J., R. Weinberg, Y. Kawaoka, R Webster and E. Paoletti, Vaccine 6, 504-508 (1988c).
24. Taylor, J., C. Edbauer, A. Rey-Senelonge, J. F. Bouquet, E. Norton, S. Goebel, P. Desmettre and E. Paoletti, J. Virol. 64, 1441-1450 (1990).
25. Taylor, J., C. Trimarchi, R. Weinberg, B. Languet, F. Guillemin, P. Desmettre and E. Paoletti, Vaccine 9,190-193 (1991).
26. Taylor J, Weinberg R, Tartaglia J, Richardson C, Alkhatib G, Briedis D, Appel M, Norton E, Paoletti E., Virology 187, 321-328 (1992).
27. Todd, D., F. D. Niagro, B. W. Ritchie, W. Curran, G. M. Allan, P. D. Lukert, K. S. Latimer, W. L. Steffens, M. S. McNulty, Arch. Virol. 117, 129-135 (1991).

Having thus described in detail preferred embodiments of the present invention, it is to be understood that the invention defined by the appended claims is not to be limited to particular details set forth in the above description as many apparent variations thereof are possible without departing from the scope of the claims.

### References

1. Allan GM, McNeilly F, Cassady JP, et al.: 1995, Pathogenesis of porcine circovirus; experimental infections of colostrum deprived piglets and examination of pig foetal material. Vet Microbiol 44: 49-641.
2. Allan GM, McNeilly F, Kennedy S, et al.: 1998, Isolation of porcine circovirus-like viruses from piglets with a wasting disease in the United States of America and Europe. J Vet Diag Invest 10 : 3-10.
3. Allan GM, Kennedy S, McNeilly F, et al.: 1999, Experimental reproduction of wasting disease and death by co-infection of pigs with porcine circovirus and porcine parvovirus, J Comp Path 121: 1-11 (July 1999).
4. Bolt DM, Hani H, Muller E, and Waldvogel AS: 1997, Nonsuppurative myocarditis in piglets associated with porcine parvovirus infection. J Comp Path 117: 107-118.
5. Ellis, JA, Hassard L, Clark EG, et al.: 1998, Isolation of circovirus from lesions of piglets with postweaning multisystemic wasting syndrome. Can Vet J 39: 44-51
6. Ellis JA, Krakowka S, Lairmore M, et al.: 1999, Reproduction of lesions of postweaning multisystemic wasting syndrome in gnotobiotic piglets. J Vet Diag Invest 11: 3-14.
7. Kim HS, Jo HS and Bergeland ME: 1989, Serologic, virologic, and histopathologic observations of encephalomyocarditis virus infection in mummified and stillborn pigs- J Vet Diag Invest 1: 101-104.
8. Lager KM and Halbur PG: 1996, Gross and microscopic lesions in porcine fetuses infected with porcine reproductive and respiratory syndrome virus. J Vet Diag Invest 8: 275-282.
9. Meehan BM, McNeilly F. Todd D, et al.: 1998, Characterization of novel circovirus DNA's associated wasting disease syndromes in pigs. J Gen Virol 79: 2171-2179.
10. Mengeling WL 1992, Porcine parvovirus. In: Diseases of swine, ed. Leman AD, 7th ed-, pp. 299-31 1. Iowa State University Press, Ames, IA.
11. Molitor TW, Orveerakul K, Zhang ZZ, et al.: 1991, Polymerase chain reaction (PRC) amplification for detection of porcine parvovirus. J Virol Meth 32: 201-211
12. Pensaert M and DeMeurichy W: 1973, A porcine enterovirus causing fetal death and mummification. Experimental infection of pregnant female pigs. Zentralbl Veterinaermed Beih 11: 2025.
13. Tischer I, Rasch R and Tochtermann G: 1974, Characterization of papovavirus-and picomavirus-like particles-in permanent piglet kidney cell lines. Zentralbl-Bakertiol-Org-A 226: 153-167.
14. West KH, Bystrom, JM, Wojnarowicz C, et al.: 1999, Myocarditis and abortion associated with intrauterine infection of sows with porcine circovirus-2. J Vet Diag Invest 1.

## Claims

1. A first DNA plasmid vector comprising a first nucleotide sequence having at least 95% identity with ORF13 of PCV-2 strain Imp1010, for use in reducing the viral load of PCV-2 in the bronchial and mesenteric nodes of a pig.

2. An immunogenic composition for use in reducing the viral load of PCV-2 in the bronchial and mesenteric nodes of a pig, wherein said composition comprises:
a) a first DNA plasmid vector comprising a first nucleotide sequence having at least 95% identity with ORF13 of PCV-2 strain Imp1010, and
b) a pharmaceutically or veterinarily acceptable carrier and/or vehicle and/or excipient and/or adjuvant.

3. Use of:
a) a first DNA plasmid vector comprising a first nucleotide sequence having at least 95% identity with ORF13 of PCV-2 strain Imp1010 and
b) a pharmaceutically or veterinarily acceptable carrier and/or vehicle and/or excipient and/or adjuvant
in the preparation of an immunogenic composition for use in reducing the viral load of PCV-2 in the bronchial and mesenteric nodes of a pig.

4. A first DNA plasmid vector comprising a first nucleotide sequence having at least 95% identity with ORF13 of PCV-2 strain Imp1010, and a second DNA plasmid vector comprising a second nucleotide sequence having at least 95% identity with ORF4 of PCV-2 strain Imp1010, for use in reducing the viral load of PCV-2 in the bronchial and mesenteric nodes of a pig.

5. An immunogenic composition for use in reducing the viral load of PCV-2 in the bronchial and mesenteric nodes of a pig, wherein said composition comprises:
a) a first DNA plasmid vector comprising a first nucleotide sequence having at least 95% identity with ORF13 of PCV-2 strain Imp1010,
b) a second DNA plasmid vector comprising a second nucleotide sequence having at least 95% identity with ORF4 of PCV-2 strain Imp1010, and
c) a pharmaceutically or veterinarily acceptable carrier and/or vehicle and/or excipient and/or adjuvant.

6. Use of:
a) a first DNA plasmid vector comprising a first nucleotide sequence having at least 95% identity with ORF13 of PCV-2 strain Imp1010,
b) a second DNA plasmid vector comprising a second nucleotide sequence having at least 95% identity with ORF4 of PCV-2 strain Imp1010, and
c) a pharmaceutically or veterinarily acceptable carrier and/or vehicle and/or excipient and/or adjuvant
in the preparation of an immunogenic composition for use in reducing the viral load of PCV-2 in the bronchial and mesenteric nodes of a pig.

7. The first plasmid for use according to claim 1; or the immunogenic composition for use according to claim 2; or the use of the first plasmid according to claim 3; or the first and second plasmids for use according to claim 4; or the immunogenic composition for use according to claim 5; or the use of first and second plasmids according to claim 6; wherein said first nucleotide sequence is ORF13 of PCV-2 strain Imp1010.

8. The first and second plasmids for use according to claim 4 or 7; or the immunogenic composition for use according to claim 5 or 7; or the use of first and second plasmids according to claim 6 or 7; wherein said second nucleotide sequence is ORF4 of PCV-2 strain Imp1010.

## Patentansprüche

1. Ein erster DNA-Plasmid-Vektor, umfassend eine erste Nucleotidsequenz, welche mindestens eine 95%-ige Identität zu ORF13 des PCV-2-Stamms Imp1010 hat, zur Verwendung in der Reduktion der Viruslast von PCV-2 in den bronchialen und mesenterialen Knoten eines Schweins.

2. Immunogene Zusammensetzung zur Verwendung in der Reduktion der Viruslast von PCV-2 in den bronchialen und mesenterialen Knoten eines Schweins, wobei die Zusammensetzung umfasst:
(a) einen ersten DNA-Plasmid-Vektor, umfassend eine erste Nucleotidsequenz, welche mindestens eine 95%-ige Identität zu ORF13 des PCV-2-Stamms Imp1010 hat, und
(b) einen pharmazeutisch oder tierärztlich verträglichen Träger und/oder Vehikel und/oder Exzipienten und/oder Adjuvans.

3. Verwendung
(a) eines ersten DNA-Plasmid-Vektors, umfassend eine erste Nucleotidsequenz, welche mindestens eine 95%-ige Identität zu ORF13 des PCV-2-Stamms Imp1010 hat, und
(b) eines pharmazeutisch oder tierärztlich verträglichen Trägers und/oder Vehikels und/oder Exzipienten und/oder Adjuvans,
in der Herstellung einer immunogenen Zusammensetzung zur Verwendung in der Reduktion der Viruslast von PCV-2 in den bronchialen und mesenterialen Knoten eines Schweins.

4. Ein erster DNA-Plasmid-Vektor, umfassend eine erste Nucleotidsequenz, welche mindestens eine 95%-ige Identität zu ORF13 des PCV-2-Stamms Imp1010 hat, und ein zweiter DNA-Plasmid-Vektor, umfassend eine zweite Nucleotidsequenz, welche mindestens eine 95%-ige Identität zu ORF4 des PCV-2-Stamms Imp1010 hat, zur Verwendung in der Reduktion der Viruslast von PCV-2 in den bronchialen und mesenterialen Knoten eines Schweins.

5. Immunogene Zusammensetzung zur Verwendung in der Reduktion der Viruslast von PCV-2 in den bronchialen und mesenterialen Knoten eines Schweins, wobei die Zusammensetzung umfasst:
(a) einen ersten DNA-Plasmid-Vektor, umfassend eine erste Nucleotidsequenz, welche mindestens eine 95%-ige Identität zu ORF13 des PCV-2-Stamms Imp1010 hat,
(b) einen zweiten DNA-Plasmid-Vektor, umfassend eine zweite Nucleotidsequenz, welche mindestens eine 95%-ige Identität zu ORF4 des PCV-2-Stamms Imp1010 hat, und
(c) einen pharmazeutisch oder tierärztlich verträglichen Träger und/oder Vehikel und/oder Exzipienten und/oder Adjuvans.

6. Verwendung
(a) eines ersten DNA-Plasmid-Vektors, umfassend eine erste Nucleotidsequenz, welche mindestens eine 95%-ige Identität zu ORF13 des PCV-2-Stamms Imp1010 hat,
(b) eines zweiten DNA-Plasmid-Vektors, umfassend eine zweite Nucleotidsequenz, welche mindestens eine 95%-ige Identität zu ORF4 des PCV-2-Stamms Imp1010 hat, und
(c) eines pharmazeutisch oder tierärztlich verträglichen Trägers und/oder Vehikels und/oder Exzipienten und/oder Adjuvans,
in der Herstellung einer immunogenen Zusammensetzung zur Verwendung in der Reduktion der Viruslast von PCV-2 in den bronchialen und mesenterialen Knoten eines Schweins.

7. Das erste Plasmid zur Verwendung gemäß Anspruch 1; oder die immunogene Zusammensetzung zur Verwendung gemäß Anspruch 2; oder die Verwendung des ersten Plasmids gemäß Anspruch 3; oder das erste und zweite Plasmid zur Verwendung gemäß Anspruch 4; oder die immunogene Zusammensetzung zur Verwendung gemäß Anspruch 5; oder die Verwendung von ersten und zweiten Plasmiden gemäß Anspruch 6; wobei die erste Nucleotidsequenz ORF13 des PCV-2-Stamms Imp1010 ist.

8. Das erste und zweite Plasmid zur Verwendung gemäß Anspruch 4 oder 7; oder die immunogene Zusammensetzung zur Verwendung gemäß Anspruch 5 oder 7; oder die Verwendung von ersten oder zweiten Plasmiden gemäß Anspruch 6 oder 7; wobei die zweite Nucleotidsequenz ORF4 des PCV-2-Stamms Imp1010 ist.

## Revendications

1. Premier vecteur plasmidique à ADN comprenant une première séquence nucléotidique ayant au moins 95 % d'identité avec l'ORF13 de la souche Imp1010 de PCV-2, destiné à être utilisé dans la réduction de la charge virale de PCV-2 dans les ganglions bronchiques et mésentériques d'un porc.

2. Composition immunogène destinée à être utilisée dans la réduction de la charge virale de PCV-2 dans les ganglions bronchiques et mésentériques d'un porc, où ladite composition comprend :
a) un premier vecteur plasmidique à ADN comprenant une première séquence nucléotidique ayant au moins 95 % d'identité avec l'ORF13 de la souche Imp1010 de PCV-2, et
b) un vecteur et/ou véhicule et/ou excipient et/ou adjuvant acceptable du point de vue pharmaceutique ou vétérinaire.

3. Utilisation de :
a) un premier vecteur plasmidique à ADN comprenant une première séquence nucléotidique ayant au moins 95 % d'identité avec l'ORF13 de la souche Imp1010 de PCV-2 et
b) un vecteur et/ou véhicule et/ou excipient et/ou adjuvant acceptable du point de vue pharmaceutique ou vétérinaire
dans la préparation d'une composition immunogène destinée à être utilisée dans la réduction de la charge virale de PCV-2 dans les ganglions bronchiques et mésentériques d'un porc.

4. Premier vecteur plasmidique à ADN comprenant une première séquence nucléotidique ayant au moins 95 % d'identité avec l'ORF13 de la souche Imp 1010 de PCV-2 et second vecteur plasmidique à ADN comprenant une seconde séquence nucléotidique ayant au moins 95 % d'identité avec l'ORF4 de la souche Imp1010 de PCV-2, destinés à être utilisés dans la réduction de la charge virale de PCV-2 dans les ganglions bronchiques et mésentériques d'un porc.

5. Composition immunogène destinée à être utilisée dans la réduction de la charge virale de PCV-2 dans les ganglions bronchiques et mésentériques d'un porc, où ladite composition comprend :
a) un premier vecteur plasmidique à ADN comprenant une première séquence nucléotidique ayant au moins 95 % d'identité avec l'ORF13 de la souche Imp1010 de PCV-2,
b) un second vecteur plasmidique à ADN comprenant une seconde séquence nucléotidique ayant au moins 95 % d'identité avec l'ORF4 de la souche Imp1010 de PCV-2, et
c) un vecteur et/ou véhicule et/ou excipient et/ou adjuvant acceptable du point de vue pharmaceutique ou vétérinaire.

6. Utilisation de :
a) un premier vecteur plasmidique à ADN comprenant une première séquence nucléotidique ayant au moins 95 % d'identité avec l'ORF13 de la souche Imp1010 de PCV-2 et
b) un second vecteur plasmidique à ADN comprenant une seconde séquence nucléotidique ayant au moins 95 % d'identité avec l'ORF4 de la souche Imp1010 de PCV-2, et
un vecteur et/ou véhicule et/ou excipient et/ou adjuvant acceptable du point de vue pharmaceutique ou vétérinaire
dans la préparation d'une composition immunogène destinée à être utilisée dans la réduction de la charge virale de PCV-2 dans les ganglions bronchiques et mésentériques d'un porc.

7. Premier plasmide destiné à être utilisé selon la revendication 1 ; ou composition immunogène destinée à être utilisée selon la revendication 2 ; ou utilisation du premier plasmide selon la revendication 3 ; ou premier et second plasmides destinés à être utilisés selon la revendication 4 ; ou composition immunogène destinée à être utilisée selon la revendication 5 ; ou utilisation de premier et second plasmides selon la revendication 6 ; où ladite première séquence nucléotidique est l'ORF13 de la souche Imp1010 de PCV-2.

8. Premier et second plasmides destinés à être utilisés selon la revendication 4 ou 7 ; ou composition immunogène destinée à être utilisée selon la revendication 5 ou 7 ; ou utilisation de premier et second plasmides selon la revendication 6 ou 7 ; où ladite seconde séquence nucléotidique est l'ORF4 de la souche Imp1010 de PCV-2.
